# EUROPEAN PATENT APPLICATION

(11) **EP 2 107 375 A1**
(43) Date of publication of application: **07.10.2009**
(21) Application number: 08153813.4
(22) Date of filing: 31.03.2008
(51) Int. Cl.: G01N 33/574

(54) **Uses of WARS protein in cancer prognostics**

(71) Applicant: Atlas Antibodies AB, 106 91 Stockholm (SE)
(72) Inventor: Pontén, Fredrik, 752 37 Uppsala (SE); Uhlén, Mathias, 182 79 Stocksund (SE); Jirström, Karin, 216 18 Limhamn (SE)
(74) Representative: Henriksson, Dan Ragnar Mikael

(57) **Abstract**

The invention provides new methods, means and uses for establishing a prognosis for mammalian subjects having a colorectal cancer. The provided method for determining whether a prognosis for the mammalian subject is worse than or equal to a reference prognosis comprises the steps of: providing a sample earlier obtained from the subject; evaluating the amount of WARS protein present in at least part of the sample, and determining a sample value corresponding to the evaluated amount; comparing the obtained sample value with a reference value associated with the reference prognosis; and, if the sample value is equal to or lower than the reference value, concluding that the prognosis for the subject is equal to or worse than the reference prognosis.

SEQUENCE LISTING

<110>  Atlas Antibodies

<120>  Uses of WARS protein in cancer prognostics

<130>  21035794

<160>  3

<170>  PatentIn version 3.3

<210>  1
<211>  144
<212>  PRT
<213>  Homo sapiens

<400>  1

Asp Ala Thr Glu Ala Glu Glu Asp Phe Val Asp Pro Trp Thr Val Gln
1               5                   10                  15

Thr Ser Ser Ala Lys Gly Ile Asp Tyr Asp Lys Leu Ile Val Arg Phe
            20                  25                  30

Gly Ser Ser Lys Ile Asp Lys Glu Leu Ile Asn Arg Ile Glu Arg Ala
        35                  40                  45

Thr Gly Gln Arg Pro His His Phe Leu Arg Arg Gly Ile Phe Phe Ser
    50                  55                  60

His Arg Asp Met Asn Gln Val Leu Asp Ala Tyr Glu Asn Lys Lys Pro
65                  70                  75                  80

Phe Tyr Leu Tyr Thr Gly Arg Gly Pro Ser Ser Glu Ala Met His Val
                85                  90                  95

Gly His Leu Ile Pro Phe Ile Phe Thr Lys Trp Leu Gln Asp Val Phe
            100                 105                 110

Asn Val Pro Leu Val Ile Gln Met Thr Asp Asp Glu Lys Tyr Leu Trp
        115                 120                 125

Lys Asp Leu Thr Leu Asp Gln Ala Tyr Ser Tyr Ala Val Glu Asn Ala
    130                 135                 140

<210>  2
<211>  430
<212>  PRT
<213>  Homo sapiens

<400>  2

Met Leu Val Ser Leu Lys Met Ser Tyr Lys Ala Ala Ala Gly Glu Asp
1               5                   10                  15

Tyr Lys Ala Asp Cys Pro Pro Gly Asn Pro Ala Pro Thr Ser Asn His
            20                  25                  30

Gly Pro Asp Ala Thr Glu Ala Glu Glu Asp Phe Val Asp Pro Trp Thr
        35                  40                  45

Val Gln Thr Ser Ser Ala Lys Gly Ile Asp Tyr Asp Lys Leu Ile Val
    50                  55                  60

Arg Phe Gly Ser Ser Lys Ile Asp Lys Glu Leu Ile Asn Arg Ile Glu
65                  70                  75                  80

Arg Ala Thr Gly Gln Arg Pro His His Phe Leu Arg Arg Gly Ile Phe
                85                  90                  95

Phe Ser His Arg Asp Met Asn Gln Val Leu Asp Ala Tyr Glu Asn Lys
            100                 105                 110

Lys Pro Phe Tyr Leu Tyr Thr Gly Arg Gly Pro Ser Ser Glu Ala Met
        115                 120                 125

His Val Gly His Leu Ile Pro Phe Ile Phe Thr Lys Trp Leu Gln Asp
    130                 135                 140

Val Phe Asn Val Pro Leu Val Ile Gln Met Thr Asp Asp Glu Lys Tyr
145                 150                 155                 160

Leu Trp Lys Asp Leu Thr Leu Asp Gln Ala Tyr Ser Tyr Ala Val Glu
                165                 170                 175

Asn Ala Lys Asp Ile Ile Ala Cys Gly Phe Asp Ile Asn Lys Thr Phe
            180                 185                 190

Ile Phe Ser Asp Leu Asp Tyr Met Gly Met Ser Ser Gly Phe Tyr Lys
        195                 200                 205

Asn Val Val Lys Ile Gln Lys His Val Thr Phe Asn Gln Val Lys Gly
    210                 215                 220

Ile Phe Gly Phe Thr Asp Ser Asp Cys Ile Gly Lys Ile Ser Phe Pro
225                 230                 235                 240

Ala Ile Gln Ala Ala Pro Ser Phe Ser Asn Ser Phe Pro Gln Ile Phe
                245                 250                 255

Arg Asp Arg Thr Asp Ile Gln Cys Leu Ile Pro Cys Ala Ile Asp Gln
            260                 265                 270

Asp Pro Tyr Phe Arg Met Thr Arg Asp Val Ala Pro Arg Ile Gly Tyr
        275                 280                 285

Pro Lys Pro Ala Leu Leu His Ser Thr Phe Phe Pro Ala Leu Gln Gly
    290                 295                 300

Ala Gln Thr Lys Met Ser Ala Ser Asp Pro Asn Ser Ser Ile Phe Leu
305                 310                 315                 320

Thr Asp Thr Ala Lys Gln Ile Lys Thr Lys Val Asn Lys His Ala Phe
                325                 330                 335

Ser Gly Gly Arg Asp Thr Ile Glu Glu His Arg Gln Phe Gly Gly Asn
            340                 345                 350

Cys Asp Val Asp Val Ser Phe Met Tyr Leu Thr Phe Phe Leu Glu Asp
        355                 360                 365

Asp Asp Lys Leu Glu Gln Ile Arg Lys Asp Tyr Thr Ser Gly Ala Met
    370                 375                 380

Leu Thr Gly Glu Leu Lys Lys Ala Leu Ile Glu Val Leu Gln Pro Leu
385                 390                 395                 400

Ile Ala Glu His Gln Ala Arg Arg Lys Glu Val Thr Asp Glu Ile Val
                405                 410                 415

Lys Glu Phe Met Thr Pro Arg Lys Leu Ser Phe Asp Phe Gln
            420                 425                 430

<210>  3
<211>  2712
<212>  DNA
<213>  Homo sapiens

<400>  3
tcgattctca agagggtttc attggtctca acctggcccc ccaggcaacc cacccctgat      60

tggacagtct catcaagaag gttggtcaag agctcaagtg tttctgagaa tctgggtgat     120

ttataagaaa cccttagctg aatgcagggt ggggagaacg aaagacaaaa gcatcttttt     180

tcagaaggga aactgaaaga aagaggggaa gagtattaaa gaccatttct ggctgggcag     240

ggcactctca gcagctcaac tgcccagcgt gaccagtggc cacctctgca gtgtcttcca     300

caacctggga tgaaattgat tctgcagtaa agatgttggt gtcattaaaa atgagctaca     360

aagctgccgc gggggaggat tacaaggctg actgtcctcc agggaaccca gcacctacca     420

gtaatcatgg cccagatgcc acagaagctg aagaggattt tgtggaccca tggacagtac     480

agacaagcag tgcaaaaggc atagactacg ataagctcat tgttcggttt ggaagtagta     540

aaattgacaa agagctaata aaccgaatag agagagccac cggccaaaga ccacaccact     600

tcctgcgcag aggcatcttc ttctcacaca gagatatgaa tcaggttctt gatgcctatg     660

aaaataagaa gccattttat ctgtacacgg gccggggccc ctcttctgaa gcaatgcatg     720

taggtcacct cattccattt attttcacaa agtggctcca ggatgtattt aacgtgccct     780

tggtcatcca gatgacggat gacgagaagt atctgtggaa ggacctgacc ctggaccagg     840

cctatagcta tgctgtggag aatgccaagg acatcatcgc ctgtggcttt gacatcaaca     900

agactttcat attctctgac ctggactaca tggggatgag ctcaggtttc tacaaaaatg     960

tggtgaagat tcaaaagcat gttaccttca accaagtgaa aggcattttc ggcttcactg 1020
acagcgactg cattgggaag atcagttttc ctgccatcca ggctgctccc tccttcagca 1080
actcattccc acagatcttc cgagacagga cggatatcca gtgccttatc ccatgtgcca 1140
ttgaccagga tccttacttt agaatgacaa gggacgtcgc ccccaggatc ggctatccta 1200
aaccagccct gctgcactcc accttcttcc cagccctgca gggcgcccag accaaaatga 1260
gtgccagcga ccccaactcc tccatcttcc tcaccgacac ggccaagcag atcaaaacca 1320
aggtcaataa gcatgcgttt tctggaggga gagacaccat cgaggagcac aggcagtttg 1380
ggggcaactg tgatgtggac gtgtctttca tgtacctgac cttcttcctc gaggacgacg 1440
acaagctcga gcagatcagg aaggattaca ccagcggagc catgctcacc ggtgagctca 1500
agaaggcact catagaggtt ctgcagccct tgatcgcaga gcaccaggcc cggcgcaagg 1560
aggtcacgga tgagatagtg aaagagttca tgactccccg gaagctgtcc ttcgactttc 1620
agtagcactc gttttacata tgcttataaa agaagtgatg tatcagtaat gtatcaataa 1680
tcccagccca gtcaaagcac cgccacctgt aggcttctgt ctcatggtaa ttactgggcc 1740
tggcctctgt aagcctgtgt atgttatcaa tactgtttct tcctgtgagt tccattattt 1800
ctatctctta tgggcaaagc attgtgggta attggtgctg gctaacattg catggtcgga 1860
tagagaagtc cagctgtgag tctctcccca aagcagcccc acagtggagc ctttggctgg 1920
aagtccatgg gccaccctgt tcttgtccat ggaggactcc gagggttcca agtatactct 1980
taagacccac tctgtttaaa aatatatatt ctatgtatgc gtatatggaa ttgaaatgtc 2040
attattgtaa cctagaaagt gctttgaaat attgatgtgg ggaggtttat tgagcacaag 2100
atgtatttca gcccatgccc cctcccaaaa agaaattgat aagtaaaagc ttcgttatac 2160
atttgactaa gaaatcaccc agctttaaag ctgcttttaa caatgaagat tgaacagagt 2220
tcagcaattt tgattaaatt aagacttggg ggtgaaactt tccagtttac tgaactccag 2280
accatgcatg tagtccactc cagaaatcat gctcgcttcc cttggcacac cagtgttctc 2340
ctgccaaatg accctagacc ctctgtcctg cagagtcagg gtggcttttc ccctgactgt 2400
gtccgatgcc aaggagtcct ggcctccgca gatgcttcat tttgaccctt ggctgcagtg 2460
gaagtcagca cagagcagtg ccctggctgt gtccctggac gggtggactt agctagggag 2520
aaagtcgagg cagcagccct cgaggccctc acagatgtct aggcaggcct catttcatca 2580
cgcagcatgt gcaggcctgg aagagcaaag ccaaatctca gggaagtcct tggttgatgt 2640
atctgggtct cctctggagc actctgccct cctgtcaccc agtagagtaa ataaacttcc 2700
ttggctcctg ct 2712

## Description

### Field of the invention

The present invention relates to the field of cancer prognostics. In particular, it provides new means for establishing a prognosis for a patient having a colorectal cancer.

### Background of the invention

### Cancer

Cancer is one of the most common causes of disease and death in the western world. In general, incidence rates increase with age for most forms of cancer. As human populations continue to live longer, due to an increase of the general health status, cancer will affect an increasing number of individuals. The cause of most common cancer types is still at large unknown, although there is an increasing body of knowledge providing a link between environmental factors (dietary, tobacco smoke, UV radiation etc) as well as genetic factors (germ line mutations in "cancer genes" such as p53, APC, BRCA1, XP etc) and the risk for development of cancer.

No definition of cancer is entirely satisfactory from a cell biological point of view, despite the fact that cancer is essentially a cellular disease and defined as a transformed cell population with net cell growth and anti-social behavior. Malignant transformation represents the transition to a malignant phenotype based on irreversible genetic alterations. Although this has not been formally proven, malignant transformation is believed to take place in one cell, from which a subsequently developed tumor originates (the "clonality of cancer" dogma). Carcinogenesis is the process by which cancer is generated and is generally accepted to include multiple events that ultimately lead to growth of a malignant tumor. This multi-step process includes several rate-limiting steps, such as addition of mutations and possibly also epigenetic events, leading to formation of cancer following stages of precancerous proliferation. The stepwise changes involve accumulation of errors (mutations) in vital regulatory pathways that determine cell division, asocial behavior and cell death. Each of these changes may provide a selective Darwinian growth advantage compared to surrounding cells, resulting in a net growth of the tumor cell population. It is important to emphasize that a malignant tumor does not only consist of the transformed tumor cells themselves but also surrounding normal cells which act as a supportive stroma. This recruited cancer stroma consists of connective tissue, blood vessels and various other normal cells, e.g. inflammatory cells, which act in concert to supply the transformed tumor cells with signals necessary for continued tumor growth.

The most common forms of cancer arise in somatic cells and are predominantly of epithelial origin, e.g. prostate, breast, colon, urothelial and skin, followed by cancers originating from the hematopoetic lineage, e.g. leukemia and lymphoma, neuroectoderm, e.g. malignant gliomas, and soft tissue tumors, e.g. sarcomas.

### Cancer diagnostics and prognostics

Microscopic evaluation of a tissue section taken from a tumor remains the golden standard for determining a diagnosis of cancer. For microscopic diagnosis, biopsy material from suspected tumors is collected and examined under the microscope. To obtain a firm diagnosis, the tumor tissue is fixated in formalin, histo-processed and paraffin embedded. From the resulting paraffin block, tissue sections can be produced and stained using both histochemical, i.e. hematoxylin-eosin staining, and immunohistochemical methods. The surgical specimen is then evaluated with pathology techniques, including gross and microscopic analysis. This analysis forms the basis for assigning a specific diagnosis, i.e. classifying the tumor type and grading the degree of malignancy, of a tumor.

Malignant tumors can be categorized into several stages according to classification schemes specific for each cancer type. The most common classification system for solid tumors is the tumor-node-metastasis (TNM) staging system. The T stage describes the local extent of the primary tumor, i.e. how far the tumor has invaded and imposed growth into surrounding normal tissues, whereas the N stage and M stage describe how the tumor has developed into metastasis, with the N stage describing spread of tumor to lymph nodes and the M stage describing growth of tumor in other distant organs. Early stages include: T0-1, N0, M0, representing localized tumors with negative lymph nodes. More advanced stages include: T1-4, N0-4, M0, localized tumors with more widespread growth and T1-4, N1-4, M0, tumors that have metastasized to lymph nodes and T1-4, N1-4, M1, tumors with a metastasis detected in a distant organ. Staging of tumors is often based on several forms of examinations, including surgical, radiological and histopathological analyses. In addition to the staging, there is also a classification system to grade the level of malignancy for most tumor types. The grading systems rely on morphological assessment of a tumor tissue sample and are based on the microscopic features found in a given tumor. These grading systems may be based on the degree of differentiation, proliferation and atypical appearance of the tumor cells. Examples of generally employed grading systems include Gleason grading for prostatic carcinomas and Elston-Ellis grading for breast carcinomas.

Accurate staging and grading is crucial for a correct diagnosis and provides an instrument to predict a prognosis. The diagnostic and prognostic information for a specific tumor subsequently determines an adequate therapeutic strategy for a given cancer patient. The most commonly used method, in addition to histochemical staining of tissue sections, to obtain more information regarding a tumor is immunohistochemical staining (IHC). IHC allows for the detection of protein expression patterns in tissues and cells using specific antibodies. The use of IHC in clinical diagnostics allows for the detection of immunoreactivity in different cell populations, in addition to the information regarding tissue architecture and cellular morphology that is assessed from the histochemically stained tumor tissue section. IHC can be important to support the accurate diagnosis, including staging and grading, of a primary tumor as well as in the diagnostics of metastases of unknown origin. The most commonly used antibodies in clinical practice today include antibodies against cell type "specific" proteins, e.g. PSA (prostate), MelanA (melanocytes), Thyroglobulin (thyroid gland) and antibodies recognizing intermediate filaments (epithelial, mesenchymal, glial) cluster of differentiation (CD) antigens (hematopoetic, sub-classification of lympoid cells) and markers of malignant potential, e.g. Ki67 (proliferation), p53 (commonly mutated tumor suppressor gene) and HER-2 (growth factor receptor).

Aside from IHC, the use of *in situ* hybridization for detecting gene amplification and gene sequencing for mutation analysis are evolving technologies within cancer diagnostics. In addition, global analysis of transcripts, proteins or metabolites all add important information. However, most of these analyses still represent basic research and have yet to be evaluated and standardized for the use in clinical medicine.

In order for physicians to give a cancer patient the right type of treatment as early as possible, the provision of new molecular markers that allows for more accurate stratification of cancer patients into different risk categories is crucial. We are still far a way from this type of individually tailored treatment. In summary, there is a great demand for new means to advance prognostics and staging of cancer.

### Adenocarcinomas from colon and rectum (colorectal cancer)

Colorectal cancer, a malignant epithelial tumor that presents as an adenocarcinoma, is one of the most common forms of human cancer worldwide. Data from the GLOBOCAM 2002 database presented by Parkin *et al* show that around 1 million new cases of colorectal cancer are identified yearly (Parkin DM et al (2005) CA Cancer J Clin 55, 74-108). Further, the incidence of colorectal cancer in the world is approximately 9.4 % of all cancers, and colorectal cancer constitutes the second most common cause of death in the western world. The five-year survival rate of colorectal cancer is approximately 60 % in the western world but as low as 30 % in Eastern Europe and India.

Early detection and surgery with excision of the tumor is currently of critical importance for a successful treatment. For localized tumors, i.e. tumors that have not evolved into a metastasizing disease, surgical intervention with radical resection of the tumor and surrounding bowel and tissues is performed. Colorectal tumors are categorized into several stages according to Dukes' stages A-D or more recently according to the TNM classification. The least malignant tumors (Dukes' stages A and B) are generally associated with a relatively favorable outcome, while highly malignant tumors with metastasis (Dukes' stage C and D) have poor survival rates. Unfortunately, colorectal cancer has often grown to a considerable size before detection and thus metastases are not uncommon. The tumor typically metastasizes to regional lymph nodes, but distant metastasis to the liver and lung are also common.

Symptoms depend on where in the distal gastrointestinal tract the tumor is located, and include bowel distress, diarrhea, constipation, pain and anemia (secondary to bleeding from the tumor into the bowel). Current diagnostics are based on patient history, clinical and endoscopic examination (rectoscopy and colonoscopy), optionally followed by radiological mapping to determine extensiveness of tumor growth. In conjunction with endoscopic examination, tissue biopsies are performed from dubious lesions.

In differential diagnostics, cytokeratin 20 (CK20), an intermediate filament marker abundant in the glandular cells of the GI-tract, is commonly used to diagnose primary tumors in the GI-tract including colorectal cancer. The CK20 marker is not ideal as several other adenocarcinomas also can be positive for CK20 antibodies, whereas not all colorectal cancers are positive. Prognostic information is mainly obtained from tumor staging classification as there are no accepted grading systems or protein markers that provide additional prognostic data. Today there are no available markers that can distinguish tumors of low malignancy grade and low risk for developing into a metastasizing disease from highly malignant tumors with a reduced chance of survival. There is thus a great need for molecular markers that can be used to predict patient outcome and to guide for patient management including therapeutic intervention.

### WARS

Tryptophanyl-tRNA synthetase (WARS) is a member of the aminoacyl-tRNA synthetases, a family of enzymes that are important for the cell's protein- synthesizing machinery. Several studied have shown up-regulation of the transcript in response to interferon treatment (Rubin BY et al (1991) J Biol Chem 25(266)24245-8, Fleckner J et al (1991) PNAS 88:11520-4). Interferon is a cytokine known to induce genes associated with angiogenesis (Beatty G and Paterson Y (2001) J Immunol. 166(4):2276-82). More recent studied have reviled that splice variants of WARS acts as inhibitors of angiogenesis. (Tzima E and Schimmel P (2006) Trends Biochem Sci 1:7-10).

### Disclosure of the invention

However, none of the above-mentioned studies describe tumor specific expression of WARS protein in colorectal cancer. Further, neither the relation between WARS protein expression and disease outcome, nor any prognostic relevance of the WARS protein is previously disclosed.

Summarizing the state of the art, there is a great need for new tools for colorectal cancer prognostics, i.e. the branch of medical science dealing with prognoses for subjects having colorectal cancer.

It is an object of the present invention to meet this demand through the provision of improvements relating to colorectal cancer prognostics.

For this and other objects apparent to the skilled person from the present disclosure, the present invention provides, in its different aspects, new means for determining a prognosis for a subject having a colorectal cancer, and for the treatment thereof. The present invention is defined by the appending claims.

Thus, according to a first aspect, the present invention provides a method for determining whether a prognosis for a mammalian subject having or suspected of having a colorectal cancer is worse than or equal to a reference prognosis, comprising the steps of:
a) providing a sample earlier obtained from the subject;
b) evaluating the amount of WARS protein present in at least part of said sample, and determining a sample value corresponding to said evaluated amount;
c) comparing the sample value obtained in step b) with a reference value associated with said reference prognosis; and, if said sample value is equal to or lower than said reference value,
d) concluding that the prognosis for said subject is equal to or worse than said reference prognosis.

According to an alternative embodiment of the first aspect there is provided a method for determining a prognosis for a mammalian subject having a colorectal cancer, comprising the steps of:
a) providing a sample earlier obtained from the subject;
b) evaluating the amount of WARS protein present in at least part of said sample, and determining a sample value corresponding to said evaluated amount;
c) comparing the sample value obtained in step b) with a reference value associated with a reference prognosis; and, if said sample value is equal to or lower than said reference value,
d) concluding that the prognosis for said subject is equal to or worse than said reference prognosis.

In an embodiment, any one of the above methods may comprise the additional step:
e) if the sample value is higher than the reference value, concluding that the prognosis for the subject is better than the reference prognosis.

According to another alternative embodiment of the first aspect there is provided a method for determining whether a prognosis for a mammalian subject having a colorectal cancer is: worse than or equal to a reference prognosis; or better than the reference prognosis,
the method comprising the steps of:
a) providing a sample earlier obtained from the subject;
b) evaluating the amount of WARS protein present in at least part of said sample, and determining a sample value corresponding to said evaluated amount;
c) comparing the sample value obtained in step b) with a reference value associated with a reference prognosis; and
d1) if said sample value is equal to or lower than said reference value, concluding that the prognosis for said subject is equal to or worse than said reference prognosis, or
d2) if said sample value is higher than said reference value, concluding that the prognosis for said subject is better than said reference prognosis.

Regarding step b) of the above methods, an increase in the amount of WARS protein typically results in an increase in the sample value, and not the other way around.

This first aspect of the present invention is based on the previously unrecognized fact that the amount of WARS protein present in samples earlier obtained from a subject having a colorectal cancer may serve as a disease status indicator in the subject. More particularly, the present invention identifies for the first time, in subjects suffering from a colorectal cancer, a correlation between an amount of WARS protein on the one hand and a prognosis for survival on the other. Typically, low WARS protein values have been shown to correlate with a poor prognosis in these subjects, probably due to a more aggressive or high-risk form of the cancer. The present invention based on WARS protein expression as a colorectal cancer status indicator has a number of benefits. In general, early identification of aggressive forms of colorectal cancer is of vital importance as it helps a physician selecting an appropriate treatment strategy. Also, by identifying less aggressive forms at an early stage, over-treatment may be avoided. As a further example, the WARS protein, as a marker for which a certain level of expression is correlated with a certain pattern of disease progression, has a great potential for example in a panel for differential diagnostics of a primary tumor.

In the present disclosure, different WARS values (sample values) corresponding to various prognoses are presented. Typically, a low sample value is associated with a poorer prognosis than a high sample value. In the above method, the sample value is compared to a reference value, and if the sample value is equal to, or lower than, the reference value, it is concluded that the prognosis for the subject is equal to, or worse than, a reference prognosis associated with the reference value.

Consequently, the above method may be adapted to a reference value. In such case, starting from a given sample value which under certain circumstances is considered to be relevant, a reference value which is equal to, or higher than, the given sample value, may be selected. Subsequently, a reference prognosis being associated with that reference value may be established. Guided by the present disclosure, the person skilled in the art understands how to establish a reference prognosis which corresponds to a given reference value. For example, the relation between sample values and survival data in a group of cancer patients may be examined as in Examples, Section 4, below, and the procedure described therein may be adapted to a given reference value. Then, a prognosis corresponding to the given reference value may be selected as the reference prognosis.

Also, the above method may be adapted to a given reference prognosis. In such case, starting from a given reference prognosis which under certain circumstances is considered to be relevant, for example for selecting an appropriate therapy, a corresponding reference value may be established. Guided by the present disclosure, the person skilled in the art understands how to establish a reference value which corresponds to a given reference prognosis. For example, the relation between sample values and survival data in a group of cancer patients may be examined as in Examples, Section 4, below, but the procedure described therein is adapted to establish reference values corresponding to a given reference prognosis. For example, different reference values may be tested until one which correlates with the given reference prognosis is found.

Accordingly, in embodiments of the methods of the above aspect, the reference prognosis may be based on a previously established prognosis, e.g. obtained by an examination of the same subject or population of subjects. Also, the reference prognosis may be adapted to a background risk in the general population, a statistical prognosis/risk or an assumption based on an examination of the subject. Such examination may comprise the subject's age, general condition, sex, race and/or medical status and history, such as cancer history or colorectal cancer status. For example, a physician may adapt the reference prognosis to the subject's cancer history, the stage of the tumor, the morphology of the tumor, the location of the tumor, the presence and spread of metastases and/or further cancer characteristics.

According to yet another alternative embodiment of the first aspect, there is provided a method for establishing a prognosis for a mammalian subject having a colorectal cancer:
a) providing a sample from the subject;
b) evaluating the amount of WARS protein present in at least part of the sample, and determining a sample value corresponding to the evaluated amount;
c) correlating the sample value of step b) to the prognosis for the subject.

Regarding step b), an increase in the amount of WARS protein typically results in an increase in the sample value, and not the other way around.

In the context of the present disclosure, "establishing a prognosis " refers to establishing a specific prognosis or a prognosis interval.

In an embodiment of the above method, the sample may be an earlier obtained sample.

The correlating of step c) refers to any way of associating survival data to the obtained sample value so as to establish a prognosis for the subject.

The identified correlation between low WARS expression and high-risk forms of colorectal cancer may also form the basis for applying a different regime for treatment of the subject than would have been the case if the subject had not exhibited a low WARS expression value. Thus, according to another aspect of the present disclosure, there is provided a method of treatment of a subject being in need thereof, wherein the subject is having a colorectal cancer, comprising:
a) providing a sample from the subject;
b) evaluating the amount of WARS protein present in at least part of said sample, and determining a sample value corresponding to said evaluated amount;
c) comparing the sample value obtained in step b) with a reference value; and, if said sample value is equal to or lower than said reference value,
d) treating said subject with an adjuvant colorectal cancer treatment regimen.

Regarding step b) of the above method, an increase in the amount of WARS protein typically results in an increase in the sample value, and not the other way around.

In an embodiment of the second aspect, the method may comprise the additional step:
e) and if said sample value is higher than said reference value, refraining from treating said subject with the adjuvant colorectal cancer treatment regimen.

In one embodiment of the second aspect, the reference value of step c) may be associated with a reference prognosis and said colorectal cancer treatment regimen of step d) may be adapted to a prognosis which is worse than or equal to the reference prognosis. In such an embodiment, the method may comprise the additional step: e) and if said sample value is higher than said reference value, treating said subject with a treatment regimen adapted to a prognosis which is better than the reference prognosis.

As an example, the treatment regimen of the second aspect may be selected from chemotherapy, neo-adjuvant therapy and combinations thereof.

Thus, the treatment regimen may be neo-adjuvant therapy. Such neo-adjuvant therapy may consist of radiation therapy only or radiation therapy in combination with chemotherapy.

Further, the treatment may be chemotherapy.

In general, when deciding on a suitable treatment strategy for a patient having colorectal cancer, the physician responsible for the treatment may take several parameters into account, such as the result of an immunohistochemical evaluation, patient age, hormone receptor status, general condition and medical history, such as colorectal cancer history. To be guided in such decision, the physician may perform WARS protein test, or order a WARS protein test performed, according to an embodiment of one of the method aspects presented above.

In the context of the present disclosure, "prognosis" refers to the prediction of the course or outcome of a disease and its treatment. For example, prognosis may also refer to a determination of chance of survival or recovery from a disease, as well as to a prediction of the expected survival time of a subject. A prognosis may, specifically, involve establishing the likelihood for survival of a subject during a period of time into the future, such as three years, five years, ten years or any other period of time.

In the context of the inventive methods of present disclosure, "earlier obtained" refers to obtained before the inventive method is performed. Consequently, if a sample earlier obtained from a subject is provided in a method, the method does not involve obtaining the sample from the subject, i.e. the sample was previously obtained from the subject in a step separate from the method.

Further, in the context of the present disclosure, "a mammalian subject having a colorectal cancer" refers to a mammalian subject having a primary or secondary colorectal tumor or a mammalian subject which has had a tumor removed from the colon and/or rectum, wherein the removal of the tumor refers to killing or removing the tumor by any appropriate type of surgery or therapy. In the method and use aspects of the present disclosure, "a mammalian subject having a colorectal cancer" also includes the cases wherein the mammalian subject is suspected of having a colorectal at the time of the performance of the use or method and the colorectal cancer diagnosis is established later.

Still further, in the context of the present disclosure, the "reference value" refers to a predetermined value found to be relevant for making decisions, or drawing conclusions, regarding the prognosis, or a suitable treatment strategy, for the subject.

Also, in the context of the present disclosure, a reference value being "associated" with a reference prognosis refers to the reference value being assigned a corresponding reference prognosis, based on empirical data and/or clinically relevant assumptions.

Step b) of the methods of the above aspects involve evaluating the amount of WARS protein present in at least part of the sample, and determining a sample value corresponding to the amount. The "at least part of the sample" refers to a relevant part, or relevant parts, of the sample for establishing the prognosis or drawing conclusions regarding suitable treatments. The person skilled in the art understands which part or parts that are relevant under the circumstances present when performing the method. For example, if evaluating at a sample comprising cells, the skilled person may only consider the tumor cells, or only the cytoplasms of tumor cells, of the sample.

Further, in step b) an amount is evaluated and a sample value corresponding to the amount is determined. Consequently, an exact measurement of the amount of WARS protein is not required for obtaining the sample value. For example, the amount of WARS protein may be evaluated by visual inspection of a stained tissue sample and the sample value may then be categorized as a e.g. high or low based on the evaluated amount.

The person skilled in the art understands how to perform such evaluation and determination. For example, the evaluation and determination may be performed as described below in connection with "quantification of WARS protein expression in a sample".

In embodiments of the methods of the above aspects, the reference prognosis may for example be a reference probability of survival, such as five-year survival, ten-year survival or 15-year survival. As a further example, the survival may be an overall survival. Accordingly, the prognosis for said subject of the methods of the above aspect may also be a probability of survival, such as five-year survival, ten-year survival or 15-year survival, wherein the survival may for example be an overall survival. Consequently, the prognosis being worse than or equal to the reference prognosis may correspond to a probability of survival which is lower than or equal to a reference probability of survival.

In embodiments of the methods of the above aspects, the sample is a body fluid sample. For example, the body fluid sample may be selected from the group consisting of blood, plasma, serum, cerebral fluid, urine, semen and exudate. Alternatively, the sample may be a cytology sample or a stool sample.

In further embodiments of the methods of the above aspects, the sample may be a tissue sample, such as a colorectal tissue sample, e.g. a sample derived from the colon or rectum.

In complementing embodiments of the methods of the above aspects, the sample may be a tumor tissue sample.

In embodiments of the methods of the above aspects, the sample may comprise glandular cells from said subject. Consequently, in addition to tissue samples, e.g. a stool sample or blood sample may also comprise such cells, which expression of WARS protein may be evaluated.

The inventors have found that WARS is expressed in the cytoplasm of relevant cells. Consequently, the evaluation of WARS expression in a sample may be limited to an analysis of cytoplasmic expression in tumor cells present in the sample, e.g. an earlier obtained tumor tissue biopsy material or specimen from a surgical removal of a colorectal cancer. As an example, the evaluation of step b) of the above methods may be limited to evaluating the amount WARS protein in the cytoplasms of tumor cells, such as cells originating from epithelial cells, e.g. glandular cells, of said sample.

In embodiments of the methods of the above aspects, the subject may have, or be suspected of having, colorectal cancer in different forms and/or stages.

In some embodiments of these aspects, the colorectal cancer in question is a node-negative colorectal cancer, i.e. colorectal cancer that has not progressed to the lymph node metastazing stage. In other similar embodiments, the colorectal cancer in question is characterized as being in either Dukes' stage A or B. In yet other embodiments, the colorectal cancer in question is colorectal adenoma or colorectal carcinoma. In these embodiments, determining that the subject exhibits low WARS expression may be of great value for the prognosis of future progression of the disease and thus form the basis for an informed decision with regard to future disease management. Within a group of subjects afflicted with such a comparatively early stage of disease, subjects with low WARS expression probably are at a comparatively high risk of developing a more aggressive disease. Low WARS expression among subjects having node-negative colorectal cancer or Dukes' stage A or B colorectal cancer may therefore indicate that these subjects should be monitored more closely and/or treated differently than subjects that do not exhibit low WARS expression. The methods according to the invention therefore offers the possibility of a greater chance for survival over a certain period of time and/or longer survival time for such subjects, owing to the additional prognostic information given by the WARS marker. Subjects having a Dukes' stage A colorectal cancer are traditionally not treated with adjuvant chemotherapy. However, guided by the teachings of the present disclosure, a physician may decide to give such a subject having low, or absent, WARS protein expression such an adjuvant chemotherapy.

Consequently, in embodiments of the methods of the above aspects, the colorectal cancer is in Dukes' stage A. In an alternative or complementary embodiment, said colorectal cancer is in T1-2, N0 and M0 according to the TNM staging system described above.

In other embodiments, the colorectal cancer in question is metastazing colorectal cancer. In other similar embodiments, the colorectal cancer in question is characterized as being in Dukes' stage C.

A sample value of WARS protein being higher than the reference value, or a subject from which such sample value is obtained, is sometimes referred to herein as "WARS protein high". Further, a sample value of WARS protein being lower than, or equal to, the reference value, or a subject from which such sample value is obtained, is sometimes referred to herein as "WARS protein low".

In the context of the present disclosure, the terms "sample value" and "reference value" are to be interpreted broadly. The quantification of WARS protein to obtain these values may be done via automatic means, via a scoring system based on visual or microscopic inspection of samples, or via combinations thereof. However, it is also possible for a skilled person, such as a person skilled in the art of histopathology, to determine the sample and reference values merely by inspection, e.g. of tissue slides that have been stained for WARS protein expression. The determination of the sample value being lower than or equal to the reference value may thus correspond to the determination, upon visual or microscopic inspection, that a sample tissue slide is less densely stained and/or exhibit a smaller fraction of stained cells than is the case for a reference tissue slide. The sample value may also be compared to a reference value given by a literal reference, such as a reference value described in wording. In this case, the sample and reference values are thought of as mental values that the skilled person determines upon inspection and comparison.

The skilled person may categorize a sample as being WARS protein high or low, wherein the sample is categorized as high if it contains more WARS protein than a previously inspected reference sample and low if it contains less or equally much. Such evaluation may be assisted by staining the sample, and, if necessary, a reference sample, with a staining solution comprising e.g. antibodies selective for WARS protein.

A reference value found to be relevant for the provision of a prognosis for a subject having a colorectal cancer, or for making treatment decisions regarding such subjects, for use as comparison with the sample value from the subject, may be provided in various ways. With the knowledge of the teachings of the present disclosure, the skilled artisan can, without undue burden, provide relevant reference values for performing the methods of the above aspects.

The person performing the methods of the above aspects may, for example, adapt the reference value to desired prognostic information. For example, the reference value may be adapted to yield the most significant prognostic information, e.g. the largest separation between the WARS protein high survival curve and the WARS protein low survival curve (see the figures). Examples of reference values that may yield a large separation is a cytoplasmic fraction (CF) of < 2 % or an absent cytoplasmic intensity (CI). An "absent" CI is defined below.

Alternatively, the reference value may be adapted to identify a group of subjects having a predetermined prognosis, e.g. the group of subjects having a probability of five year overall survival of less than 70 %.

In embodiments of the methods of the above aspects, the reference value may correspond to the amount of WARS protein expression in a healthy tissue, such as healthy colorectal or stroma tissue, of the subject of the method. As another example, the reference value may be provided by the amount of WARS protein expression measured in a standard sample of normal tissue from another, comparable subject. As another example, the reference value may be provided by the amount of WARS protein expression measured in a reference sample comprising tumor cells, such as a reference sample of tumor tissue. As another example, the reference value may be provided by the amount of WARS protein measured in a reference sample comprising cells expressing a predetermined amount of WARS protein.

As another example, the reference value may be provided by the amount of WARS protein expression measured in a reference sample comprising cell lines, such as cancer cell lines, expressing a predetermined, or controlled, amount of WARS protein. The person skilled in the art understands how to provide such cell lines, for example guided by the disclosure of Rhodes et al. (2006) The biomedical scientist, p 515-520.

Consequently, in embodiments of the methods of the above aspects, the reference value may be a predetermined value corresponding to the amount of WARS protein expression in a reference sample.

The inventors have shown that subjects suffering from colorectal cancer, who have lost essentially all their WARS protein expression, generally have a poor prognosis. Thus, in embodiments of the methods of the above aspects, the sample value of step b) may be either 1, corresponding to detectable WARS protein in the sample, or 0, corresponding to no detectable WARS protein in the sample. Consequently, in such embodiment, the evaluation of the sample is digital: WARS protein is considered to be either present or not. In the context of the present disclosure, "no detectable WARS protein" refers to an amount of WARS protein that is so small that it is not, during normal operational circumstances, detectable by a person or an apparatus performing the invention according to any one of its different aspects. The "normal operational circumstances" refer to the laboratory methods and techniques a person skilled in the art would find appropriate for performing the invention.

Further, in embodiments of the methods of the above aspects, the reference value of step c) may be provided by a reference sample, such as a tissue sample, having no detectable WARS protein expression.

In embodiments of the methods of the above aspects, the reference value of step c) may be 0, corresponding to no detectable WARS protein.

One alternative for the quantification of WARS protein expression in a sample, such as the sample earlier obtained from the subject or the reference sample, is the determination of the fraction of cells in the sample that exhibit WARS protein expression over a certain level. The fraction may for example be: a "cellular fraction", wherein the WARS protein expression of the whole cells is taken into account; a "cytoplasmic fraction", wherein the WARS protein expression of only the cytoplasms of the cells is taken into account, or the "nuclear fraction", wherein the WARS protein expression of only the nuclei of the cells is taken into account. The cytoplasmic fraction may for example be classified as < 2 %, 2 - 25 %, > 25 - 75 % or > 75 % immunoreactive cells of the relevant cell population. This determination may for example be performed as described below in the Examples, Section 4, with reference to "cytoplasmic fraction". The "cytoplasmic fraction" corresponds to the percentage of relevant cells in a sample that exhibits a positive staining in the cytoplasm, wherein a medium or distinct and strong immunoreactivity in the cytoplasm is considered positive and no or faint immunoreactivity in the cytoplasm is considered negative. The person skilled in the art of pathology understands which cells that are relevant under the conditions present when performing the method and may determine a cytoplasmic fraction based on his general knowledge and the teachings of the present disclosure. The relevant cells may for example be tumor cells. Further, the skilled artisan understands how to perform corresponding measurements employing the "cellular fraction" or the "nuclear fraction".

Another alternative for the quantification of WARS expression in a sample, such as the sample earlier obtained from the subject or the reference sample, is the determination of the overall staining intensity of the sample. The intensity may for example be: a "cellular intensity", wherein the WARS protein expression of the whole cells is taken into account; a "cytoplasmic intensity", wherein the WARS protein expression of only the cytoplasms of the cells is taken into account, or the "nuclear intensity", wherein the WARS protein expression of only the nuclei of the cells is taken into account. Cytoplasmic intensity is subjectively evaluated in accordance to standards used in clinical histopathological diagnostics. Outcome of a cytoplasmic intensity determination may be classified as: absent = no overall immunoreactivity in the cytoplasm of relevant cells of the sample, weak = faint overall immunoreactivity in the cytoplasm of relevant cells of the sample, moderate = medium overall immunoreactivity in the cytoplasm of relevant cells of the sample, or strong = distinct and strong overall immunoreactivity in the cytoplasm of relevant cells of the sample. The person skilled in the art understands which cells that are relevant under the conditions present when performing the method and may determine a cytoplasmic intensity based on his general knowledge and the teachings of the present disclosure. The relevant cells may for example be tumor cells. This determination may for example be performed as described below in the Examples, Section 4, definition of "cytoplasmic intensity". Further, the skilled artisan understands how to perform corresponding measurements employing the "cellular intensity" or the "nuclear intensity".

The inventors have found that the cytoplasmic expression of WARS protein is particularly relevant for establishing a prognosis. Thus, in embodiments of the methods of the above aspects, the reference value may be a cytoplasmic fraction, a cytoplasmic intensity or a combination thereof. Accordingly, the sample value may be a cytoplasmic fraction, a cytoplasmic intensity or a combination thereof.

Preferably, the sample value and the reference value are both the same type of value. Accordingly, in embodiments of the methods of the above aspects, the sample value and the reference value may each be a cytoplasmic fraction, a cytoplasmic intensity or a combinations thereof.

In embodiments of the methods of the above aspects, the criterion for the conclusion in step d) is a sample value for the cytoplasmic fraction of WARS protein positive cells, i.e. a "cytoplasmic fraction", which is lower than or equal to the reference value of 75 %, such as lower than or equal to 50 %, such as lower than or equal to 40 %, such as lower than or equal to 30 %, such as lower than or equal to 25 %, such as lower than or equal to 20 %, such as lower than or equal to 15 %, such as lower than or equal to 10 %, such as lower than or equal to 5 %, such as lower than 2 %, such as equal to 0%.

In alternative or complementing embodiments of the methods of the above aspects, the reference value of step c) is a cytoplasmic fraction of 75 % WARS positive cells or lower, such as 50 % WARS positive cells or lower, such as 40 % WARS positive cells or lower, such as 30 % WARS positive cells or lower, such as 25 % WARS positive cells or lower, such as 20 % WARS positive cells or lower, such as 10 % WARS positive cells or lower, such as 5 % WARS positive cells or lower, such as < 2 % WARS positive cells, such as 0 % WARS positive cells.

In Examples, Section 4 below, a cytoplasmic fraction of < 2 % was found to give significant results regarding prognoses. Consequently, in embodiments of the methods of the above aspects, the reference value of step c) is a cytoplasmic fraction of 0 - 25 %, such as 0 - 20 %, such as 0 - 15 %, such as 0 - 10 %, such as 0 - 5 %, such as 0 - < 2 %.

Further, in embodiments of the methods of the above aspects, the criterion for the conclusion in step d) may be a sample value for staining intensity of a sample, i.e. a cytoplasmic intensity, which is equal to, or lower than, a moderate cytoplasmic intensity, such as equal to, or lower than, a weak cytoplasmic intensity, such as equal to an absent cytoplasmic intensity. In alternative or complementing embodiments of the methods of the above aspects, the reference value of step c) may be a moderate cytoplasmic intensity of WARS protein expression, or lower, such as a weak cytoplasmic intensity of WARS protein expression, or lower, such as an absent cytoplasmic intensity of WARS protein expression.

Further, in embodiments of the methods of the above aspects, the reference value may be constituted of two values, wherein the criterion for the conclusion in step d) is a sample value being lower than or equal to any one of these two values.

Alternatively, in embodiments of the methods of the above aspects, the reference value may be a combination of a fraction value and an intensity value, such as a cytoplasmic fraction value and a cytoplasmic intensity value.

Also, in embodiments of the methods of the above aspects, the reference value may be a function of a cytoplasmic fraction value and a cytoplasmic intensity value. For example, such a function may be a staining score. The "staining score" is calculated as described in Examples, Section 3 and table 1 below. For example, the reference value may be a staining score of 2 or lower, such as 1 or lower, such as 0.

The person skilled in the art realizes that other reference values being an intensity value or a fraction value also fall within the scope of the present invention. Likewise, the person skilled in the art realizes that other combinations of fractions and intensities also fall within the scope of the present invention. Consequently, the reference value may involve two, and possibly even more, criteria.

Guided by the present disclosure, and especially Examples, Sections 3-5 below, a person skilled in the art, e.g. a pathologist, understands how to perform the evaluation yielding a fraction, such as a cellular, cytoplasmic or nuclear fraction, or an intensity, such as a cellular, cytoplasmic or nuclear intensity. For example, the skilled artisan may use a reference sample comprising a predetermined amount of WARS protein for establishing the appearance of a certain fraction or intensity.

However, a reference sample may not only be used for the provision of the actual reference value, but also for the provision of an example of a sample with an amount of WARS protein that is higher than the amount corresponding to the reference value. As an example, in histochemical staining, such as in immunohistochemical staining, the skilled artisan may use a reference sample for establishing the appearance of a stained sample with a high amount of WARS protein, e.g. a positive reference. Subsequently, the skilled artisan may assess the appearances of samples with lower amounts of WARS, such as the appearance of a sample with an amount of WARS corresponding to the reference value. In other words, the skilled artisan may use a reference sample to create a mental image of a reference value corresponding to an amount of WARS protein which is lower than that of the reference sample. Alternatively, or as a complement, in such assessments, the skilled artisan may use another reference sample having a low amount of WARS protein, or essentially lacking WARS protein, for establishing the appearance of such sample, e.g. as a "negative reference".

Consequently, in the evaluation, the skilled artisan may use a reference sample for establishing the appearance of a sample with a high amount of WARS protein. Such reference sample may be a sample comprising tissue expressing a high amount of WARS protein, such as a sample comprising colorectal tumor tissue having a pre-established high expression of WARS protein.

Accordingly, the reference sample may provide an example of a strong cytoplasmic intensity (CI). With the knowledge of the appearance of a sample with strong CI, the skilled artisan may then divide samples into the CI categories presented in Examples, Sections 3 and 4, below, i.e. absent, weak, moderate and strong. This division may be further assisted by a reference sample essentially lacking WARS protein (negative reference), i.e. a reference sample providing an absent cytoplasmic intensity. Also, the reference sample may provide an example of a sample with a cytoplasmic fraction (CF) of 75 % or higher. With the knowledge of the appearance of a sample with more than 75 % positive cells, the skilled artisan may then evaluate the cytoplasmic fraction of other samples having e.g. a lower percentage of positive cells. This division may be further assisted by a reference sample essentially lacking WARS protein (negative reference), i.e. a reference sample providing a low CF (e.g. < 5%, such as < 2%), or a CF of 0.

As mentioned above, cell lines expressing a controlled amount of WARS protein may be used as the reference, in particular as a positive reference.

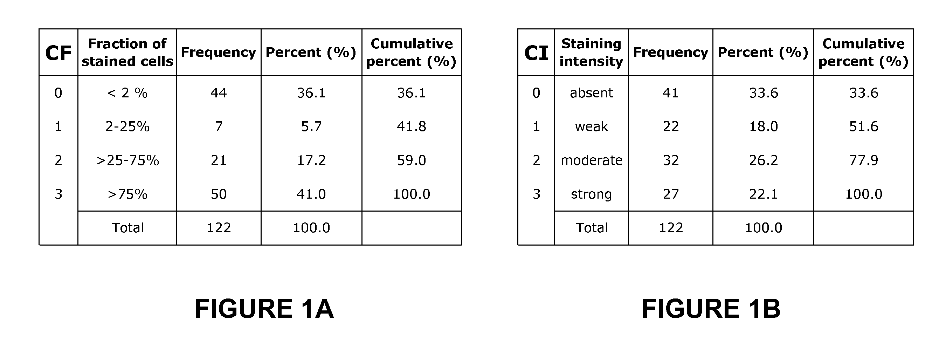

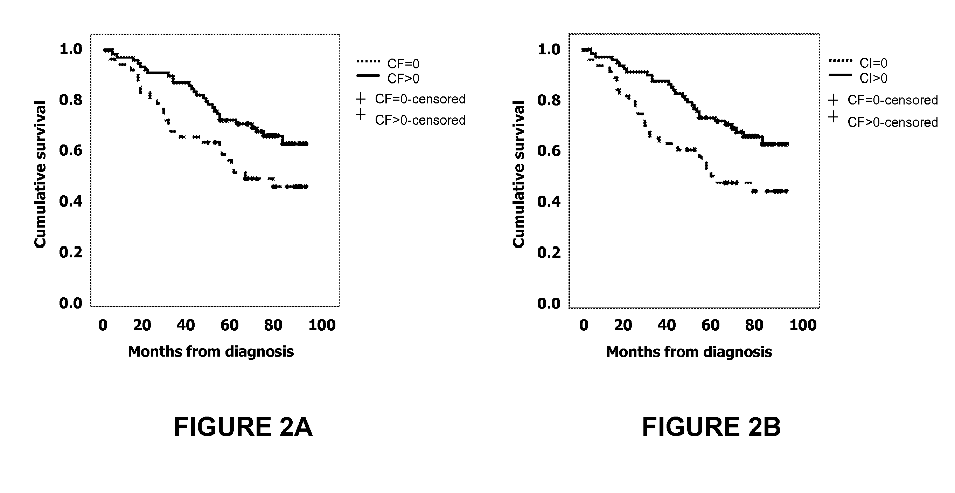

As discussed above, the methods according to the above aspects may be adapted to a selected reference value, such as one of the reference values presented above, and the reference prognosis will in such case be a consequence of the selected reference value. As a non-limiting example, if one of the reference value of Examples, Section 4 is used, CF < 2 %, an associated reference prognosis may be derived from figure 2A by looking at the CF > 0 curve (solid line), i.e. the curve representing the patients having sample values of CF = 2 % or higher. At a given time from diagnosis, the corresponding cumulative survival according to the CF > 0 curve may be read from the figure, e.g. 70 % after 5 years (60 months), which results in a reference prognosis being a probability of five-year survival of lower than 70 %. Consequently, patients of the same group having sample values which are lower than or equal to the reference value CF < 2 % have a probability of five year survival which is lower than 70 %. Using the same logic, the associated reference prognosis is a probability of five-year survival of lower than 70 % if using an absent cytoplasmic intensity (CI = 0) as the reference value (fig 1 B). Other associated reference prognoses may also be derived from the figures. The skilled artisan understands how to identify reference prognoses associated with reference values, and may do so without undue burden.

Consequently, in embodiments of the methods of the above aspects, the reference prognosis may be a likelihood of five-year survival of lower than 70 %.

Further, in embodiments of the methods of the above aspects, the reference prognosis may be a predetermined average probability of five-year survival for subjects having colorectal cancer, such as the average five-year survival for subjects having colorectal cancer at the time of performing the method. In such embodiments, the reference value is adapted to the reference prognosis, as discussed above. For example, the average five-year survival of a patient diagnosed with colorectal cancer was about 60 % world wide in 2002 (Parkin et al (2007) CA Cancer J Clin 55, 74-108). However, increased knowledge, development of new therapeutic agents and improved diagnostics will eventually increase the chance for prolonged survival for these subjects. Consequently, due to the progression of the medical art, the survival numbers above may be higher in the future, such as in the second or third decade of the 21^{st} century.

In embodiments of the methods of the above aspects, the subject may be a human.

In embodiments of the methods of the above aspects, the subject may have a cancer.

The skilled person will recognize that the usefulness of the present invention is not limited to the quantification of any particular variant of the WARS protein present in the subject in question, as long as the protein is encoded by the relevant gene and presents the relevant pattern of expression. As a non-limiting example, the WARS protein has an amino acid sequence which comprises a sequence selected from:
i) SEQ ID NO:1; and
ii) a sequence which is at least 85 % identical to SEQ ID NO:1.

In some embodiments, sequence ii) above is at least 90 % identical, at least 91 % identical, at least 92 % identical, at least 93 % identical, at least 94 % identical, at least 95 % identical, at least 96 % identical, at least 97 % identical, at least 98 % identical or at least 99 % identical to SEQ ID NO:1.

As another non-limiting example, the WARS protein has an amino acid sequence which comprises a sequence selected from:
i) SEQ ID NO:2; and
ii) a sequence which is at least 85 % identical to SEQ ID NO:2.

In some embodiments, sequence ii) above is at least 90 % identical, at least 91 % identical, at least 92 % identical, at least 93 % identical, at least 94 % identical, at least 95 % identical, at least 96 % identical, at least 97 % identical, at least 98 % identical or at least 99 % identical to SEQ ID NO:2.

In embodiments of the methods of the aspects above, the WARS protein may be detected and/or quantified through the application to a sample of a detectable and/or quantifiable affinity ligand, which is capable of selective interaction with the WARS protein. The application of the affinity ligand is performed under conditions that enable binding of the affinity ligand to any WARS protein in the sample.

To concretize, in embodiments of the methods of the aspects above, step b) may comprise:
b1) applying to the sample a quantifiable affinity ligand capable of selective interaction with the WARS protein to be evaluated, said application being performed under conditions that enable binding of the affinity ligand to any WARS protein present in the sample;
b2) removing non-bound affinity ligand; and
b3) quantifying any affinity ligand remaining in association with the sample to evaluate said amount.

"Affinity ligand remaining in association with the sample" refers to affinity ligand which was not removed in step b2), e.g. the affinity ligand bound to the sample.

It is regarded as within the capabilities of those of ordinary skill in the art to select or manufacture the proper affinity ligand and to select the proper format and conditions for detection and/or quantification, once the connection between WARS protein and a prognosis for colorectal cancer is known through the teaching of the present disclosure. Nevertheless, examples of affinity ligands that may prove useful, as well as examples of formats and conditions for detection and/or quantification, are given below for the sake of illustration.

Thus, in some embodiments of the methods of the above aspects, an affinity ligand is used, which is selected from the group consisting of antibodies, fragments thereof and derivatives thereof, i.e. affinity ligands based on an immunoglobulin scaffold. For example, the antibodies may be isolated and/or mono-specific. For example, antibodies comprise monoclonal and polyclonal antibodies of any origin, including murine, rabbit, human and other antibodies, as well as chimeric antibodies comprising sequences from different species, such as partly humanized antibodies, e.g. partly humanized mouse antibodies. Polyclonal antibodies are produced by immunization of animals with the antigen of choice, whereas monoclonal antibodies of defined specificity can be produced using the hybridoma technology developed by Köhler and Milstein (Köhler G and Milstein C (1976) Eur. J. Immunol. 6:511-519). Antibody fragments and derivatives comprise Fab fragments, consisting of the first constant domain of the heavy chain (CH1), the constant domain of the light chain (CL), the variable domain of the heavy chain (VH) and the variable domain of the light chain (VL) of an intact immunoglobulin protein; Fv fragments, consisting of the two variable antibody domains VH and VL (Skerra A and Plückthun A (1988) Science 240:1038-1041); single chain Fv fragments (scFv), consisting of the two VH and VL domains linked together by a flexible peptide linker (Bird RE and Walker BW (1991) Trends Biotechnol. 9:132-137); Bence Jones dimers (Stevens FJ et al (1991) Biochemistry 30:6803-6805); camelid heavy-chain dimers (Hamers-Casterman C et al (1993) Nature 363:446-448) and single variable domains (Cai X and Garen A (1996) Proc. Natl. Acad. Sci. U.S.A. 93:6280-6285; Masat L et al (1994) Proc. Natl. Acad. Sci. U.S.A. 91:893-896), and single domain scaffolds like e.g. the New Antigen Receptor (NAR) from the nurse shark (Dooley H et al (2003) Mol. Immunol. 40:25-33) and minibodies based on a variable heavy domain (Skerra A and Plückthun A (1988) Science 240:1038-1041).

Polyclonal and monoclonal antibodies, as well as their fragments and derivatives, represent the traditional choice of affinity ligands in applications requiring selective biomolecular recognition, such as in the detection and/or quantification of WARS protein according to the method aspects above. However, those of skill in the art know that, due to the increasing demand of high throughput generation of selective binding ligands and low cost production systems, new biomolecular diversity technologies have been developed during the last decade. This has enabled a generation of novel types of affinity ligands of both immunoglobulin as well as non-immunoglobulin origin that have proven equally useful as binding ligands in biomolecular recognition applications and can be used instead of, or together with, immunoglobulins.

The biomolecular diversity needed for selection of affinity ligands may be generated by combinatorial engineering of one of a plurality of possible scaffold molecules, and specific and/or selective affinity ligands are then selected using a suitable selection platform. The scaffold molecule may be of immunoglobulin protein origin (Bradbury AR and Marks JD (2004) J. Immunol. Meths. 290:29-49), of non-immunoglobulin protein origin (Nygren PÅ and Skerra A (2004) J. Immunol. Meths. 290:3-28), or of an oligonucleotide origin (Gold L et al (1995) Annu. Rev. Biochem. 64:763-797).

A large number of non-immunoglobulin protein scaffolds have been used as supporting structures in development of novel binding proteins. Non-limiting examples of such structures, useful for generating affinity ligands against WARS for use according to the present disclosure, are staphylococcal protein A and domains thereof and derivatives of these domains, such as protein Z (Nord K et al (1997) Nat. Biotechnol. 15:772-777); lipocalins (Beste G et al (1999) Proc. Natl. Acad. Sci. U.S.A. 96:1898-1903); ankyrin repeat domains (Binz HK et al (2003) J. Mol. Biol. 332:489-503); cellulose binding domains (CBD) (Smith GP et al (1998) J. Mol. Biol. 277:317-332; Lehtiö J et al (2000) Proteins 41:316-322); Y crystallines (Fiedler U and Rudolph R, WO01/04144); green fluorescent protein (GFP) (Peelle B et al (2001) Chem. Biol. 8:521-534); human cytotoxic T lymphocyte-associated antigen 4 (CTLA-4) (Hufton SE et al (2000) FEBS Lett. 475:225-231; Irving RA et al (2001) J. Immunol. Meth. 248:31-45); protease inhibitors, such as Knottin proteins (Wentzel A et al (2001) J. Bacteriol. 183:7273-7284; Baggio R et al (2002) J. Mol. Recognit. 15:126-134) and Kunitz domains (Roberts BL et al (1992) Gene 121:9-15; Dennis MS and Lazarus RA (1994) J. Biol. Chem. 269:22137-22144); PDZ domains (Schneider S et al (1999) Nat. Biotechnol. 17:170-175); peptide aptamers, such as thioredoxin (Lu Z et al (1995) Biotechnology 13:366-372; Klevenz B et al (2002) Cell. Mol. Life Sci. 59:1993-1998); staphylococcal nuclease (Norman TC et al (1999) Science 285:591-595); tendamistats (McConell SJ and Hoess RH (1995) J. Mol. Biol. 250:460-479; Li R et al (2003) Protein Eng. 16:65-72); trinectins based on the fibronectin type III domain (Koide A et al (1998) J. Mol. Biol. 284:1141-1151; Xu L et al (2002) Chem. Biol. 9:933-942); and zinc fingers (Bianchi E et al (1995) J. Mol. Biol. 247:154-160; Klug A (1999) J. Mol. Biol. 293:215-218; Segal DJ et al (2003) Biochemistry 42:2137-2148).

The above mentioned examples of non-immunoglobulin protein scaffolds include scaffold proteins presenting a single randomized loop used for the generation of novel binding specificities, protein scaffolds with a rigid secondary structure where side chains protruding from the protein surface are randomized for the generation of novel binding specificities, and scaffolds exhibiting a non-contiguous hyper-variable loop region used for the generation of novel binding specificities.

In addition to non-immunoglobulin proteins, oligonucleotides may also be used as affinity ligands. Single stranded nucleic acids, called aptamers or decoys, fold into well-defined three-dimensional structures and bind to their target with high affinity and specificity. (Ellington AD and Szostak JW (1990) Nature 346:818-822; Brody EN and Gold L (2000) J. Biotechnol. 74:5-13; Mayer G and Jenne A (2004) BioDrugs 18:351-359). The oligonucleotide ligands can be either RNA or DNA and can bind to a wide range of target molecule classes.

For selection of the desired affinity ligand from a pool of variants of any of the scaffold structures mentioned above, a number of selection platforms are available for the isolation of a specific novel ligand against a target protein of choice. Selection platforms include, but are not limited to, phage display (Smith GP (1985) Science 228:1315-1317), ribosome display (Hanes J and Plückthun A (1997) Proc. Natl. Acad. Sci. U.S.A. 94:4937-4942), yeast two- hybrid system (Fields S and Song O (1989) Nature 340:245-246), yeast display (Gai SA and Wittrup KD (2007) Curr Opin Struct Biol 17:467-473), mRNA display (Roberts RW and Szostak JW (1997) Proc. Natl. Acad. Sci. U.S.A. 94:12297-12302), bacterial display (Daugherty PS (2007) Curr Opin Struct Biol 17:474-480, Kronqvist N et al (2008) Protein Eng Des Sel 1-9, Harvey BR et al (2004) PNAS 101 (25):913-9198), microbead display (Nord O et al (2003) J Biotechnol 106:1-13, WO01/05808), SELEX (System Evolution of Ligands by Exponential Enrichment) (Tuerk C and Gold L (1990) Science 249:505-510) and protein fragment complementation assays (PCA) (Remy I and Michnick SW (1999) Proc. Natl. Acad. Sci. U.S.A. 96:5394-5399).

Thus, in embodiments of the methods of the above aspects, an affinity ligand may be used, which is a non-immunoglobulin affinity ligand derived from any of the protein scaffolds listed above, or an oligonucleotide molecule.

In embodiments of the methods of the above aspects, the quantifiable affinity ligand may be capable of selective interaction with a WARS protein having the sequence SEQ ID NO:1.

In some embodiments of the methods of the above aspects, an affinity ligand capable of selective interaction with the WARS protein is detectable and/or quantifiable. The detection and/or quantification of such an affinity ligand may be accomplished in any way known to the skilled person for detection and/or quantification of binding reagents in assays based on biological interactions. Thus, any affinity ligand, as described above, may be used quantitatively or qualitatively to detect the presence of the WARS protein. These "primary" affinity ligands may be labeled themselves with various markers or may in turn be detected by secondary, labeled affinity ligands to allow detection, visualization and/or quantification. This can be accomplished using any one or more of a multitude of labels, which can be conjugated to the affinity ligand capable of interaction with WARS protein or to any secondary affinity ligand, using any one or more of a multitude of techniques known to the skilled person, and not as such involving any undue experimentation.

Non-limiting examples of labels that can be conjugated to primary and/or secondary affinity ligands include fluorescent dyes or metals (e.g. fluorescein, rhodamine, phycoerythrin, fluorescamine), chromophoric dyes (e.g. rhodopsin), chemiluminescent compounds (e.g. luminal, imidazole) and bioluminescent proteins (e.g. luciferin, luciferase), haptens (e.g. biotin). A variety of other useful fluorescers and chromophores are described in Stryer L (1968) Science 162:526-533 and Brand L and Gohlke JR (1972) Annu. Rev. Biochem. 41:843-868. Affinity ligands can also be labeled with enzymes (e.g. horseradish peroxidase, alkaline phosphatase, beta-lactamase), radioisotopes (e.g. ³H, ¹⁴C, ³²P, ³⁵S or ¹²⁵I) and particles (e.g. gold). In the context of the present disclosure, "particles" refers particles, such as metal particles, suitable for labeling of molecules. Further, the affinity ligands may also be labeled with fluorescent semiconductor nanocrystals (Quantum dots). Quantum dots have superior quantum yield and are more photostable compared to organic fluorophores and are therefore more easily detected (Chan et al (2002) Curr Opi Biotech. 13: 40-46). The different types of labels can be conjugated to an affinity ligand using various chemistries, e.g. the amine reaction or the thiol reaction. However, other reactive groups than amines and thiols can be used, e.g. aldehydes, carboxylic acids and glutamine.

The method aspects above may be put to use in any of several known formats and set-ups, of which a non-limiting selection is discussed below.

In a set-up based on histology, the detection, localization and/or quantification of a labeled affinity ligand bound to its WARS protein target may involve visualizing techniques, such as light microscopy or immunofluoresence microscopy. Other methods may involve the detection via flow cytometry or luminometry.

A biological sample, such as a tumor tissue sample (biopsy), for example from colorectal tissue, may be removed from the subject for detection and/or quantification of WARS protein. Alternatively, the biological sample, such as the biopsy, may be an earlier obtained sample. If using an earlier obtained sample, no steps of any one of the embodiments of the methods of the above aspects are practiced on the human or animal body. The affinity ligand may be applied to the biological sample for detection and/or quantification of the WARS marker protein. This procedure enables not only detection of WARS protein, but may in addition show the distribution and relative level of expression thereof.

The method of visualization of labels on the affinity ligand may include, but is not restricted to, fluorometric, luminometric and/or enzymatic techniques. Fluorescence is detected and/or quantified by exposing fluorescent labels to light of a specific wavelength and thereafter detecting and/or quantifying the emitted light in a specific wavelength region. The presence of a luminescently tagged affinity ligand may be detected and/or quantified by luminescence developed during a chemical reaction. Detection of an enzymatic reaction is due to a color shift in the sample arising from chemical reaction. Those of skill in the art are aware that a variety of different protocols can be modified in order for proper detection and/or quantification.

In embodiments of the methods of the above aspects, a biological sample may be immobilized onto a solid phase support or carrier, such as nitrocellulose or any other solid support matrix capable of immobilizing any WARS protein present in the biological sample applied to it. Some well-known solid state support materials useful in the present invention include glass, carbohydrate (e.g. Sepharose), nylon, plastic, wool, polystyrene, polyethene, polypropylene, dextran, amylase, films, resins, cellulose, polyacrylamide, agarose, alumina, gabbros and magnetite. After immobilization of the biological sample, primary affinity ligand specific to WARS may be applied, e.g. as described in Examples, Sections 2 and/or 3, of the present disclosure. If the primary affinity ligand is not labeled in itself, the supporting matrix may be washed with one or more appropriate buffers known in the art, followed by exposure to a secondary labeled affinity ligand and washed once again with buffers to remove unbound affinity ligands. Thereafter, selective affinity ligands may be detected and/or quantified with conventional methods. The binding properties for an affinity ligand may vary from one solid state support to the other, but those skilled in the art will be able to determine operative and optimal assay conditions for each determination by routine experimentation.

Consequently, in embodiments of the methods of the above aspects, the quantifiable affinity ligand of b1) may be detected using a secondary affinity ligand capable of recognizing the quantifiable affinity ligand. The quantification of b3) may thus be carried out by means of a secondary affinity ligand with affinity for the quantifiable affinity ligand. As an example, the secondary affinity ligand may be an antibody or a fragment or a derivative thereof.

As an example, one available method for detection and/or quantification of the WARS protein is by linking the affinity ligand to an enzyme that can then later be detected and/or quantified in an enzyme immunoassay (such as an EIA or ELISA). Such techniques are well established, and their realization does not present any undue difficulties to the skilled person. In such methods, the biological sample is brought into contact with a solid material or with a solid material conjugated to an affinity ligand against the WARS protein, which is then detected and/or quantified with an enzymatically labeled secondary affinity ligand. Following this, an appropriate substrate is brought to react in appropriate buffers with the enzymatic label to produce a chemical moiety, which for example is detected and/or quantified using a spectrophotometer, fluorometer, luminometer or by visual means.

As stated above, primary and any secondary affinity ligands can be labeled with radioisotopes to enable detection and/or quantification. Non-limiting examples of appropriate radiolabels in the current invention are ³H, ¹⁴C, ³²P, ³⁵S or ¹²⁵I. The specific activity of the labeled affinity ligand is dependent upon the half-life of the radiolabel, isotopic purity, and how the label has been incorporated into the affinity ligand. Affinity ligands are preferably labeled using well-known techniques (Wensel TG and Meares CF (1983) in: Radioimmunoimaging and Radioimmunotherapy (Burchiel SW and Rhodes BA eds.) Elsevier, New York, pp 185-196). A thus radiolabeled affinity ligand can be used to visualize WARS protein by detection of radioactivity *in vivo* or *in vitro.* Radionuclear scanning with e.g. gamma camera, magnetic resonance spectroscopy or emission tomography function for detection *in vivo* and *in vitro,* while gamma/beta counters, scintillation counters and radiographies are also used *in vitro.*

As a further aspect of the present disclosure, there is provided a kit for carrying out a method according to an embodiment of the above aspects, which comprises:
a) a quantifiable affinity ligand capable of selective interaction with a WARS protein; and
b) reagents necessary for quantifying the amount of the affinity ligand.

As a non-limiting example, the affinity ligand is capable of selective interaction with a WARS protein having an amino acid sequence which comprises a sequence selected from:
i) SEQ ID NO:1; and
ii) a sequence which is at least 85 % identical to SEQ ID NO:1.

In some embodiments, sequence ii) above is at least 90 % identical, at least 91 % identical, at least 92 % identical, at least 93 % identical, at least 94 % identical, at least 95 % identical, at least 96 % identical, at least 97 % identical, at least 98 % identical or at least 99 % identical to SEQ ID NO:1.

As another non-limiting example, the affinity ligand is capable of selective interaction with a WARS protein having an amino acid sequence which comprises a sequence selected from:
i) SEQ ID NO:2; and
ii) a sequence which is at least 85 % identical to SEQ ID NO:2.

In some embodiments, sequence ii) above is at least 90 % identical, at least 91 % identical, at least 92 % identical, at least 93 % identical, at least 94 % identical, at least 95 % identical, at least 96 % identical, at least 97 % identical, at least 98 % identical or at least 99 % identical to SEQ ID NO:2.

Various components of the kit according to the kit aspect may be selected and specified as described above in connection with the method aspects of the present disclosure.

Thus, the kit according to the invention comprises an affinity ligand against WARS, as well as other means that help to quantify the specific and/or selective affinity ligand after it has bound specifically and/or selectively to WARS. For example, the kit may contain a secondary affinity ligand for detecting and/or quantifying a complex formed by any WARS protein and the affinity ligand capable of selective interaction with a WARS protein. The kit may also contain various auxiliary substances other than affinity ligands, to enable the kit to be used easily and efficiently. Examples of auxiliary substances include solvents for dissolving or reconstituting lyophilized protein components of the kit, wash buffers, substrates for measuring enzyme activity in cases where an enzyme is used as a label, target retrieval solution to enhance the accessibility to antigens in cases where paraffin or formalin-fixed tissue samples are used, and substances such as reaction arresters, e.g. endogenous enzyme block solution to decrease the background staining and/or counterstaining solution to increase staining contrast, that are commonly used in immunoassay reagent kits.

Thus, in embodiments of the kit aspect, the quantifiable affinity ligand is selected from the group consisting of antibodies, fragments thereof and derivatives thereof. As an example, such quantifiable affinity ligand may be obtainable by a process comprising a step of immunizing an animal, such as a rabbit, with a protein whose amino acid sequence comprises the sequence SEQ ID NO:1 or a sequence which is at least 85 % identical to SEQ ID NO:1, preferably the sequence SEQ ID NO:1. Such immunization may for example be performed as described in Examples, Section 2, below. Also, the antibodies may for example be isolated and/or mono-specific.

Alternatively, the quantifiable affinity ligand is a protein ligand derived from a scaffold selected from the group consisting of staphylococcal protein A and domains thereof, lipocalins, ankyrin repeat domains, cellulose binding domains, Y crystallines, green fluorescent protein, human cytotoxic T lymphocyte-associated antigen 4, protease inhibitors, PDZ domains, peptide aptamers, staphylococcal nuclease, tendamistats, fibronectin type III domain and zinc fingers. As a further alternative, the quantifiable affinity ligand is an oligonucleotide molecule.

The inventors have designed the sequence SEQ ID NO:1 to be a particularly suitable antigen for being recognized by the affinity ligands of the present disclosure. Consequently, in embodiments of the kit aspect, the quantifiable affinity ligand may be capable of selective interaction with a WARS protein having the sequence SEQ ID NO:1.

Further, in embodiments of the kit aspect, the detectable affinity ligand may comprise a label selected from the group consisting of fluorescent dyes and metals, chromophoric dyes, chemiluminescent compounds and bioluminescent proteins, enzymes, radioisotopes, particles and quantum dots. Alternatively, the reagents necessary for quantifying the amount of the affinity ligand comprise a secondary affinity ligand capable of recognizing the quantifiable affinity ligand. As an example, the secondary affinity ligand capable of recognizing the quantifiable affinity ligand comprises a label selected from the group consisting of fluorescent dyes or metals, chromophoric dyes, chemiluminescent compounds and bioluminescent proteins, enzymes, radioisotopes, particles and quantum dots.

The kit according to the kit aspect may also advantageously comprise a reference sample for provision of, or yielding, the reference value to be used for comparison with the sample value. Preferably, the reference sample comprises a predetermined amount of WARS protein. Such a reference sample may for example be constituted by a tissue sample having a predetermined amount of WARS protein, which may then be used by the person of skill in the art to determine the WARS expression status in the sample being studied, by manual, such as ocular, or automated comparison of expression levels in the reference sample and the subject sample. As another example, the reference sample may comprise cell lines, such as cancer cell lines, expressing a predetermined, or controlled, amount of WARS protein. The person skilled in the art understands how to provide such cell lines, for example guided by the disclosure of Rhodes et al. (2006) The biomedical scientist, p 515-520. As an example, the cell lines may be formalin fixed. Also, such formalin fixed cell lines may be paraffin embedded.

The wording "reference sample for provision of the reference value" is to be interpreted broadly in the context of the kit aspect. The reference sample may comprise an amount of WARS protein actually corresponding to the reference value, but it may also comprise an amount of WARS protein corresponding to a value being higher than the reference value. In the latter case, the "high" value may be used by a person performing the method as an upper reference (positive reference) for assessing, e.g. the appearance of, a reference value which is lower than the "high" value. The person skilled in the art of immunohistochemistry understands how to do such an assessment. Further, as an alternative or a complementing example, the skilled person may use another reference sample comprising a low amount of WARS protein for provision of a "low" value in such an assessment, e.g. as a negative reference.

Consequently, in embodiments of the kit aspect, the reference sample may comprise an amount of WARS protein corresponding to the reference value. As an example, the reference sample may comprise an amount of WARS protein corresponding to a cytoplasmic fraction of 75 % WARS positive cells or lower, such as 50 % WARS positive cells or lower, such as 40 % WARS positive cells or lower, such as 30 % WARS positive cells or lower, such as 25 % WARS positive cells or lower, such as 20 % WARS positive cells or lower, such as 10 % WARS positive cells or lower, such as 5 % WARS positive cells or lower, such as < 2 % WARS positive cells, such as 0 % WARS positive cells. Alternatively, or as a complement, the reference sample may comprise an amount of WARS protein corresponding to an moderate cytoplasmic intensity of WARS protein expression, or lower, such as a faint cytoplasmic intensity of WARS protein expression, or lower, such as no cytoplasmic intensity of WARS protein expression.

Further, the reference sample may comprise an amount of WARS protein corresponding a staining score 2 or lower, such as 1 or lower, such as 0. The provision of cytoplasmic fraction values or cytoplasmic intensity values is discussed above in connection with the method aspects.

Further, in alternative or complementing embodiments of the kit aspect, the kit may comprise a reference sample comprising an amount of WARS protein corresponding to a value being higher than the reference value. In these embodiments, the reference sample may for example comprise an amount of WARS protein corresponding to a cytoplasmic fraction of 75 % or higher and/or a strong cytoplasmic intensity of WARS expression.

In other alternative or complementing embodiments of the kit aspect, the kit may comprise a reference sample comprising an amount of WARS protein corresponding to a value being lower than the reference value, e.g. an absent cytoplasmic intensity and/or a cytoplasmic fraction of < 2 % WARS positive cells, such as 0 % WARS positive cells.

Also, in alternative or complementing embodiments of the kit aspect, the kit may comprise: a reference sample comprising an amount of WARS protein corresponding to a predetermined reference value; a reference sample comprising an amount of WARS protein corresponding to a value being higher than a predetermined reference value; and/or a reference sample comprising an amount of WARS protein corresponding to a value being lower than a predetermined reference value.

Consequently, embodiments of the kit may comprise: a first reference sample comprising an amount of WARS protein being higher than a predetermined reference value; and a second reference sample comprising an amount of WARS protein being lower than the predetermined reference value.

In embodiments of the kit aspect, the reference sample may be a tissue sample, such as a tissue sample adapted to ocular or microscopic evaluation. As an example, the tissue reference sample may be fixated in paraffin or buffered formalin and/or histo-processed to µm-thin sections that are mounted on microscopic glass-slides. The tissue reference sample may be further adapted to staining with affinity ligands, such as antibodies, for a WARS protein.

Consequently, in embodiments of the kit aspect, the reference sample may be adapted to directly, or indirectly, provide any relevant reference value, such as any one of the reference values discussed above.

Accordingly, further embodiments of the reference sample of the kit aspect are discussed above in connection with the reference values and reference samples of the method aspects.

As a further aspect of the present disclosure, there is provided the use of a WARS protein as a prognostic marker. Also provided is the use of a WARS protein as a prognostic marker for cancer, such as colorectal cancer.

In the context of the present disclosure, a "prognostic marker" refers to a product which presence is of value in an establishment of a prognosis.

The prognostic relevance of WARS protein found by the inventors entails the application of WARS protein in various assays or other laboratory set-ups. A WARS protein having the amino acid sequence of SEQ ID NO:1, which has been designed to have desired antigenic properties, may be particularly suitable for such purposes.

Accordingly, as a further use aspect of the present disclosure, there is provided the use of a WARS protein, or an antigenically active fragment thereof, for the production, selection or purification of a prognostic agent for establishing a prognosis for a patient having a colorectal cancer.

In the context of the present disclosure, an "antigenically active fragment" of a WARS protein is a fragment of sufficient size to be useful for the generation of an affinity ligand, e.g. an antibody, which will interact with a WARS protein comprising the fragment. Further, in the context of the present disclosure, a "prognostic agent" refers to an agent having at least one property being valuable in an establishment of a prognosis.

For example, the prognostic agent may be an affinity ligand capable of selective interaction with the WARS protein, or antigenically active fragment thereof. In turn, the affinity ligand may preferably be capable of selective interaction with a protein having the sequence SEQ ID NO:1. Also, the affinity ligand may preferably be an antibody or a fragment or a derivative thereof. For example, the antibody may be isolated and/or mono-specific. Guided by the teachings of the present disclosure, the person skilled in the art understands how to use WARS protein in the production, selection or purification of the prognostic agent. For example, such use may comprise affinity purification on a solid support onto which the WARS protein has been immobilized. The solid support may for example be arranged in a column. Further, the use may comprise selection of prognostic agents having specificity for the WARS protein using a soluble matrix in which the WARS protein has been immobilized. Such soluble matrix may for example be a dextran matrix for use in a surface plasmon resonance instrument, such as Biacore™ instrument and the selection may for example comprise monitoring the affinity for the immobilized WARS protein of a number of prognostic agents. Also, the use may comprise immunizing a mammal with the WARS protein in order to produce prognostic agents that are collected from the serum of the immunized mammal.

In embodiments of the above use aspects, the WARS protein may for example comprise a sequence selected from: i) SEQ ID NO:1; and ii) a sequence which is at least 85 % identical to SEQ ID NO:1. In some embodiments, sequence ii) is at least 90 % identical, at least 91 % identical, at least 92 % identical, at least 93 % identical, at least 94 % identical, at least 95 % identical, at least 96 % identical, at least 97 % identical, at least 98 % identical or at least 99 % identical to SEQ ID NO:1.

Also, the WARS protein may for example comprise a sequence selected from: i) SEQ ID NO:2; and ii) a sequence which is at least 85 % identical to SEQ ID NO:2. In some embodiments, sequence ii) is at least 90 % identical, at least 91 % identical, at least 92 % identical, at least 93 % identical, at least 94 % identical, at least 95 % identical, at least 96 % identical, at least 97 % identical, at least 98 % identical or at least 99 % identical to SEQ ID NO:2.

In embodiments of the above use aspects, the use may be *in vitro*-use.

The inventors have found a previously unknown correlation between WARS protein and the prognosis for a patient suffering from a colorectal cancer. Further, the inventors have developed an affinity ligand with affinity for the WARS protein. However, the inventive scope is not limited to a single type of affinity ligand, and the inventive idea may be put into practice using any type of affinity ligand capable of selective interaction with a WARS protein.

Thus, as a further aspect of the present disclosure, there is provided an affinity ligand capable of selective interaction with a WARS protein, such as a WARS protein having the amino acid sequence SEQ ID NO:1. In one embodiment, the affinity ligand is an antibody or a fragment or a derivative thereof. For example, the antibody may be isolated and/or mono-specific. SEQ ID NO:1 is a WARS protein especially designed for immunizations, e.g. designed to lack transmembrane regions to ensure efficient expression in *E*. *coli,* and to lack any signal peptide, since those are cleaved off in the mature protein. Consequently, the antibody, or fragment or derivative thereof, may for example be one that is obtainable by a process comprising a step of immunizing an animal, such as a rabbit, with a protein whose amino acid sequence comprises the sequence SEQ ID NO:1 or a sequence which is at least 85 % identical to SEQ ID NO:1, preferably the sequence SEQ ID NO:1. For example, the immunization process may comprise primary immunization with the protein in Freund's complete adjuvant. Also, the immunization process may further comprise boosting at least two times, in intervals of 2-6 weeks, with the protein in Freund's incomplete adjuvant. Processes for the production of antibodies or fragments or derivatives thereof against a given target are known in the art, and may be applied in connection with this aspect of the present disclosure. Any of those variants of the WARS protein (e.g. SEQ ID NO:2) or the antigenically active fragment thereof (e.g. SEQ ID NO:1) that are discussed above may, of course, be used in such a process for generating an antibody or a fragment or derivative thereof. Further, the present disclosure provides the above affinity ligand for evaluating the amount of WARS protein present in at least part of a sample obtained from a subject having a colorectal cancer. Also, the present disclosure provides the above affinity ligand for establishing a prognosis for a mammalian subject having a colorectal cancer.

As a further aspect of the present disclosure, there is provided the use of the affinity ligand according to the above aspect as a prognostic agent. A preferred embodiment of this use is as a prognostic agent for the prognosis of a cancer, such as a colorectal cancer. As a related aspect thereof, there is provided a use of the affinity ligand in the manufacture of a prognostic agent for the prognosis of a colorectal cancer. For example, the uses may be *in vitro*-uses.

In the context of the present invention, "specific" or "selective" interaction of e.g. an affinity ligand with its target or antigen means that the interaction is such that a distinction between specific and non-specific, or between selective and non-selective, interaction becomes meaningful. The interaction between two proteins is sometimes measured by the dissociation constant. The dissociation constant describes the strength of binding (or affinity) between two molecules. Typically the dissociation constant between an antibody and its antigen is from 10⁻⁷ to 10⁻¹¹ M. However, high specificity does not necessarily require high affinity. Molecules with low affinity (in the molar range) for its counterpart have been shown to be as specific as molecules with much higher affinity. In the case of the present disclosure, a specific or selective interaction refers to the extent to which a particular method can be used to determine the presence and/or amount of a specific protein, the target protein or a fragment thereof, under given conditions in the presence of other proteins in a tissue sample or fluid sample of a naturally occurring or processed biological fluid. In other words, specificity or selectivity is the capacity to distinguish between related proteins. Specific and selective are sometimes used interchangeably in the present description. For example, the specificity or selectivity of an antibody may be determined as in Examples, section 2, below, wherein analysis is performed using a protein array set-up and a western blot, respectively. Specificity and selectivity determinations are also described in Nilsson P et al (2005) Proteomics 5:4327-4337.

In the context of the present invention, a "mono-specific antibody" is one of a population of polyclonal antibodies which has been affinity purified on its own antigen, thereby separating such mono-specific antibodies from other antiserum proteins and non-specific antibodies. This affinity purification results in antibodies that bind selectively to its antigen. In the case of the present invention, the polyclonal antisera are purified by a two-step immunoaffinity based protocol to obtain mono-specific antibodies selective for the target protein. Antibodies directed against generic affinity tags of antigen fragments are removed in a primary depletion step, using the immobilized tag protein as the capturing agent. Following the first depletion step, the serum is loaded on a second affinity column with the antigen as capturing agent, in order to enrich for antibodies specific for the antigen (see also Nilsson P et al (2005) Proteomics 5:4327-4337).

### ITEMIZED LISTING OF EMBODIMENTS OF THE INVENTION

The following is a non-limiting and itemized listing of embodiments of the present invention, presented for the purpose of providing further information regarding the various features and combinations provided by the invention.
1. Method for determining whether a prognosis for a mammalian subject having a colorectal cancer is worse than or equal to a reference prognosis, comprising the steps of:
   a) providing a sample earlier obtained from the subject;
   b) evaluating the amount of WARS protein present in at least part of said sample, and determining a sample value corresponding to said evaluated amount;
   c) comparing the sample value obtained in step b) with a reference value associated with said reference prognosis; and, if said sample value is equal to or lower than said reference value,
   d) concluding that the prognosis for said subject is equal to or worse than said reference prognosis.
2. Method of treatment of a subject in need thereof, wherein the subject is having a colorectal cancer, comprising:
   a) providing a sample from the subject;
   b) evaluating the amount of WARS protein present in at least part of said sample, and determining a sample value corresponding to said evaluated amount;
   c) comparing the sample value obtained in step b) with a reference value; and, if said sample value is equal to or lower than said reference value,
   d) treating said subject with an adjuvant colorectal cancer treatment regimen.
3. Method according to item 2, wherein said reference value of step c) is associated with a reference prognosis and said colorectal cancer treatment regimen of step d) is adapted to a prognosis for said subject which is worse than or equal to the reference prognosis.
4. Method according to item 3, wherein said treatment regimen is selected from chemotherapy, neo-adjuvant therapy and combinations thereof.
5. Method according to item 4, wherein said treatment regimen is neo-adjuvant therapy.
6. Method according to item 5, wherein said neo-adjuvant therapy is selected from i) radiation therapy only and ii) radiation therapy in combination with chemotherapy.
7. Method according to any preceding item, wherein the sample is a body fluid sample.
8. Method according to item 7, wherein the body fluid is selected from the group consisting of blood, plasma, serum, cerebral fluid, urine, semen and exudate.
9. Method according to any one of items 1-6, wherein said sample is a tissue sample.
10. Method according to item 9, wherein the tissue sample is a colorectal tissue sample.
11. Method according to any one of items 1-6, wherein the sample is a cytology sample.
12. Method according to any one of items 1-6, wherein the sample is a stool sample.
13. Method according to any preceding item, wherein said sample comprises tumor cells from said subject.
14. Method according to any preceding item, wherein the evaluation of step b) is limited to evaluating the amount of WARS expression in the cytoplasm of tumor cells of said sample.
15. Method according to any preceding item, wherein said colorectal cancer is in Dukes' stage A or B.
16. Method according to any preceding item, wherein said colorectal cancer is in Dukes' stage A.
17. Method according to any preceding item, wherein said colorectal cancer is previously classified as T1-2, N0 and M0 according to the TNM staging system.
18. Method according any preceding item, wherein said prognosis for said subject is a probability of survival and said reference prognosis is a probability of survival.
19. Method according to any preceding item, wherein said reference value is a predetermined value corresponding to the amount of WARS protein expression in a reference sample.
20. Method according to any preceding item, wherein the sample value of step b) is determined as being either 1, corresponding to detectable WARS protein in the sample, or 0, corresponding to no detectable WARS protein in the sample.
21. Method according to any preceding item, wherein the reference value of step c) corresponds to a reference sample having no detectable WARS protein.
22. Method according to any preceding item, wherein the reference value of step c) is 0.
23. Method according to any preceding item, wherein said reference value is a cytoplasmic fraction, a cytoplasmic intensity or a combination thereof.
24. Method according to any preceding item, wherein said sample value and said reference value are each a cytoplasmic fraction, a cytoplasmic intensity or a combinations thereof.
25. Method according to item 24, wherein said reference value is a cytoplasmic fraction of 25 % WARS positive cells, or lower.
26. Method according to item 25, wherein said reference value is a cytoplasmic fraction of less than 2 % WARS positive cells.
27. Method according to any one of items 1-24, wherein said reference value is a weak cytoplasmic intensity of WARS protein expression, or lower.
28. Method according to item 27, wherein said reference value is an absent cytoplasmic intensity of WARS protein expression.
29. Method according to any preceding item, wherein said subject is a human.
30. Method according to any preceding item, wherein the amino acid sequence of the WARS protein comprises a sequence selected from:
   i) SEQ ID NO:1; and
   ii) a sequence which is at least 85 % identical to SEQ ID NO:1.
31. Method according to any preceding item, wherein the amino acid sequence of the WARS protein comprises a sequence selected from:
   i) SEQ ID NO:2; and
   ii) a sequence which is at least 85 % identical to SEQ ID NO:2.
32. Method according to any preceding item, wherein step b) comprises:
   b1) applying to the sample a quantifiable affinity ligand capable of selective interaction with the WARS protein to be evaluated, said application being performed under conditions that enable binding of the affinity ligand to any WARS protein present in the sample;
   b2) removing non-bound affinity ligand; and
   b3) quantifying any affinity ligand remaining in association with the sample to evaluate said amount.
33. Method according to item 32, wherein the quantifiable affinity ligand is selected from the group consisting of antibodies, fragments thereof and derivatives thereof.
34. Method according to item 32, wherein the quantifiable affinity ligand is a protein ligand derived from a scaffold selected from the group consisting of staphylococcal protein A and domains thereof, lipocalins, ankyrin repeat domains, cellulose binding domains, Y crystallines, green fluorescent protein, human cytotoxic T lymphocyte-associated antigen 4, protease inhibitors, PDZ domains, peptide aptamers, staphylococcal nuclease, tendamistats, fibronectin type III domain and zinc fingers.
35. Method according to item 32, wherein the quantifiable affinity ligand is an oligonucleotide molecule.
36. Method according to any one of items 32-35, wherein the quantifiable affinity ligand is capable of selective interaction with a WARS protein having the sequence of SEQ ID NO:1.
37. Method according to any one of items 32-36, wherein the quantifiable affinity ligand comprises a label selected from the group consisting of fluorescent dyes and metals, chromophoric dyes, chemiluminescent compounds and bioluminescent proteins, enzymes, radioisotopes, particles and quantum dots.
38. Method according to any one of items 32-37, wherein said quantifiable affinity ligand is detected using a secondary affinity ligand capable of recognizing the quantifiable affinity ligand.
39. Method according to item 38, wherein said secondary affinity ligand capable of recognizing the quantifiable affinity ligand comprises a label selected from the group consisting of fluorescent dyes and metals, chromophoric dyes, chemiluminescent compounds and bioluminescent proteins, enzymes, radioisotopes, particles and quantum dots.
40. Method according to any preceding item, wherein said reference prognosis is a five-year survival probability of less than 70 %.
41. Method according to any one of items 1-40, wherein said reference prognosis is a predetermined average probability of five-year survival for mammalian subjects having colorectal cancer.
42. Method according to any one of items 1 and 7-41, which, if said sample value is higher than said reference value, further comprises a step of:
   e) concluding that the prognosis for said subject is better than said reference prognosis.
43. Kit for carrying out the method according to any preceding item, which comprises
   a) a quantifiable affinity ligand capable of selective interaction with a WARS protein; and
   b) reagents necessary for quantifying the amount of the affinity ligand.
44. Kit according to item 43, in which the quantifiable affinity ligand is selected from the group consisting of antibodies, fragments thereof and derivatives thereof.
45. Kit according to item 44, in which the quantifiable affinity ligand is obtainable by a process comprising a step of immunizing an animal with a protein whose amino acid sequence comprises the sequence SEQ ID NO:1.
46. Kit according to item 43, in which the quantifiable affinity ligand is a protein ligand derived from a scaffold selected from the group consisting of staphylococcal protein A and domains thereof, lipocalins, ankyrin repeat domains, cellulose binding domains, Y crystallines, green fluorescent protein, human cytotoxic T lymphocyte-associated antigen 4, protease inhibitors, PDZ domains, peptide aptamers, staphylococcal nuclease, tendamistats, fibronectin type III domain and zinc fingers.
47. Kit according to item 43, in which the quantifiable affinity ligand is an oligonucleotide molecule.
48. Kit according to any one of items 43-47, in which the quantifiable affinity ligand is capable of selective interaction with a polypeptide having the sequence SEQ ID NO:1.
49. Kit according to any one of items 43-48, in which the quantifiable affinity ligand comprises a label selected from the group consisting of fluorescent dyes and metals, chromophoric dyes, chemiluminescent compounds and bioluminescent proteins, enzymes, radioisotopes, particles and quantum dots.
50. Kit according to any one of items 43-49, in which said reagents necessary for quantifying the amount of the affinity ligand comprise a secondary affinity ligand capable of recognizing the quantifiable affinity ligand.
51. Kit according to item 50, in which said secondary affinity ligand capable of recognizing the quantifiable affinity ligand comprises a label selected from the group consisting of fluorescent dyes or metals, chromophoric dyes, chemiluminescent compounds and bioluminescent proteins, enzymes, radioisotopes, particles and quantum dots.
52. Kit according to any one of items 43-51, further comprising at least one reference sample for provision of a reference value.
53. Kit according to item 52, in which at least one reference sample is a tissue sample having no detectable WARS protein present.
54. Kit according to any one of items 52-53, in which at least one reference sample comprises WARS protein.
55. Kit according to item 54, in which at least one reference sample comprises an amount of WARS protein corresponding to a cytoplasmic fraction of 25 % WARS positive cells or lower.
56. Kit according to item 55, in which at least one reference sample comprises an amount of WARS protein corresponding to a cytoplasmic fraction of less than 2 % WARS protein positive cells.
57. Kit according to item 54, in which at least one reference sample comprises an amount of WARS protein corresponding to a weak cytoplasmic intensity of WARS protein expression or lower.
58. Kit according to item 57, in which at least one reference sample comprises an amount of WARS protein corresponding to an absent cytoplasmic intensity of WARS protein expression.
59. Kit according to any one of items 52-58, in which at least one reference sample contains an amount of WARS protein corresponding to a value being higher than the reference value.
60. Kit according to item 59, in which at least one reference sample contains an amount of WARS protein corresponding to a strong cytoplasmic intensity.
61. Kit according to item 59, in which at least one reference sample contains an amount of WARS protein corresponding to a cytoplasmic fraction of 75 % WARS positive cells or higher.
62. Kit according to any one of items 52-61 comprising:
   a first reference sample comprising an amount of WARS protein being higher than the reference value; and
   a second reference sample comprising an amount of WARS protein being lower than the reference value.
63. Kit according to any one of items 52-62, in which the reference sample(s) is/are tissue sample(s).
64. Use of a WARS protein as a prognostic marker.
65. Use of a WARS protein as a prognostic marker for cancer.
66. Use according to item 65, wherein said cancer is a colorectal cancer.
67. Use of a WARS protein, or an antigenically active fragment thereof, for the production, selection or purification of a prognostic agent for establishing a prognosis for a patient having a colorectal cancer.
68. Use according to item 67, wherein said prognostic agent is an affinity ligand capable of selective interaction with the WARS protein, or an antigenically active fragment thereof.
69. Use according to item 68, wherein the affinity ligand is capable of selective interaction with a protein having the sequence SEQ ID NO:1.
70. Use according any one of items 64-69, wherein the amino acid sequence of the WARS protein comprises a sequence selected from:
   i) SEQ ID NO:1; and
   ii) a sequence which is at least 85 % identical to SEQ ID NO:1.
71. Use according any one of items 64-70, wherein the amino acid sequence of the WARS protein comprises a sequence selected from:
   i) SEQ ID NO:2; and
   ii) a sequence which is at least 85 % identical to SEQ ID NO:2.
72. Affinity ligand capable of selective interaction with a WARS protein, which is an antibody or a fragment or a derivative thereof.
73. Affinity ligand according to item 72, which is obtainable by a process comprising a step of immunizing an animal with a protein whose amino acid sequence comprises the sequence SEQ ID NO:1.
74. Affinity ligand according to item 73, which is obtainable by a process comprising a step of immunizing an animal with a polypeptide having the sequence SEQ ID NO:1.
75. Affinity ligand according to any one of items 72-74, capable of selective interaction with a polypeptide having the sequence SEQ ID NO:1.
76. Affinity ligand according to any one of items 72-75 for evaluating the amount of WARS protein present in at least part of a sample obtained from a subject having a colorectal cancer.
77. Affinity ligand according to any one of items 72-76 for establishing a prognosis for a mammalian subject having a colorectal cancer.
78. Use of an affinity ligand according to any one of items 72-77 as a prognostic agent.
79. Use of an affinity ligand according to any one of items 72-77 for establishing a prognosis for a mammalian subject having a colorectal cancer.

### Brief description of the figures

Figure 1 shows the level of immunohistochemical staining of WARS in 122 patients diagnosed with colorectal carcinomas. The subjects were scored for WARS expression. Figure 1A shows level of cytoplasmic fraction (CF). Figure 1B shows level of cytoplasmic intensity (CI).
Figure 2 shows the results of a survival analysis based on immunohistochemical staining of 122 subjects diagnosed with colorectal carcinomas. Figure 2A is based on cytoplasmic fraction. The solid line represents positive cells, i.e. fraction of stained cells ≥ 2% (CF > 0) and the dotted line represents negative cells, i.e. fraction of stained cells < 2 % (CF = 0). Figure 2B is based on cytoplasmic intensity. The solid line represents a weak, moderate or strong cytoplasmic intensity (CI > 0) and the dotted line represents an absent cytoplasmic intensity (CI = 0).

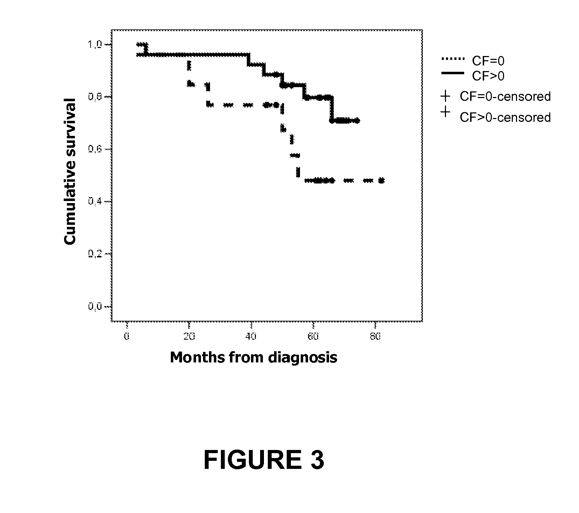

Figure 3 shows the results of a survival analysis based on immunohistochemical staining of 39 subjects diagnosed with Dukes' stage A colorectal carcinomas. The survival analysis was based on cytoplasmic intensity, wherein the solid line represents a weak, moderate or strong cytoplasmic intensity (CI > 0) and the dotted line represents an absent cytoplasmic intensity (CI = 0).

### Examples

### Generation of mono-specific antibodies against WARS and use thereof to detect WARS in normal and cancerous samples

### 1. Generation of antigen

### a) Materials and methods

A suitable fragment of the target protein encoded by the EnsEMBL Gene ID ENSG00000140105 was selected using bioinformatic tools with the human genome sequence as template (Lindskog M et al (2005) Biotechniques 38:723-727, EnsEMBL, www.ensembl.org). The fragment was used as template for the production of a 144 amino acid long fragment corresponding to amino acids 34-178 (SEQ ID NO:1) of the WARS protein (SEQ ID NO:2; EnsEMBL entry no. ENSP00000339485).

A fragment of the WARS gene transcript containing nucleotides 434-866 of EnsEMBL entry number ENST00000344102 (SEQ ID NO:3), was isolated by a Superscript™ One-Step RT-PCR amplification kit with Platinum® Taq (Invitrogen) and a human total RNA pool panel as template (Human Total RNA Panel IV, BD Biosciences Clontech). Flanking restriction sites NotI and AscI were introduced into the fragment through the PCR amplification primers, to allow in-frame cloning into the expression vector (forward primer: GATGCCACAGAAGCTGAAG, reverse primer: GGCATTCTCCACAGCATAG). Then, the downstream primer was biotinylated to allow solid-phase cloning as previously described, and the resulting biotinylated PCR product was immobilized onto Dynabeads M280 Streptavidin (Dynal Biotech) (Larsson M et al (2000) J. Biotechnol. 80:143-157). The fragment was released from the solid support by NotI-AscI digestion (New England Biolabs), ligated into the pAff8c vector (Larsson M *et al, supra*) in frame with a dual affinity tag consisting of a hexahistidyl tag for immobilized metal ion chromatography (IMAC) purification and an immunopotentiating albumin binding protein (ABP) from streptococcal protein G (Sjölander A et al (1997) J. Immunol. Methods 201:115-123; Ståhl S et al (1999) Encyclopedia of Bioprocess Technology: Fermentation, Biocatalysis and Bioseparation (Fleckinger MC and Drew SW, eds) John Wiley and Sons Inc., New York, pp 49-63), and transformed into *E. coli* BL21 (DE3) cells (Novagen). The sequences of the clones were verified by dye-terminator cycle sequencing of plasmid DNA amplified using TempliPhi DNA sequencing amplification kit (GE Healthcare, Uppsala, Sweden) according to the manufacturer's recommendations.

BL21 (DE3) cells harboring the expression vector were inoculated in 100 ml 30 g/l tryptic soy broth (Merck KGaA) supplemented with 5 g/l yeast extract (Merck KGaA) and 50 mg/l kanamycin (Sigma-Aldrich) by addition of 1 ml of an overnight culture in the same culture medium. The cell culture was incubated in a 1 liter shake flask at 37 °C and 150 rpm until the optical density at 600 nm reached 0.5-1.5. Protein expression was then induced by addition of isopropyl-β-D-thiogalactopyranoside (Apollo Scientific) to a final concentration of 1 mM, and the incubation was continued overnight at 25 °C and 150 rpm. The cells were harvested by centrifugation at 2400 *g*, and the pellet was re-suspended in 5 ml lysis buffer (7 M guanidine hydrochloride, 47 mM Na₂HPO₄, 2.65 mM NaH₂PO₄, 10 mM Tris-HCl, 100 mM NaCl, 20 mM β-mercaptoethanol; pH = 8.0) and incubated for 2 hours at 37 °C and 150 rpm. After centrifugation at 35300 g, the supernatant containing the denatured and solubilized protein was collected.

The His₆-tagged fusion protein was purified by immobilized metal ion affinity chromatography (IMAC) on columns with 1 ml Talon® metal (Co²⁺) affinity resin (BD Biosciences Clontech) using an automated protein purification procedure (Steen J et al (2006) Protein Expr. Purif. 46:173-178) on an ASPEC XL4™ (Gilson). The resin was equilibrated with 20 ml denaturing washing buffer (6 M guanidine hydrochloride, 46.6 mM Na₂HPO₄, 3.4 mM NaH₂PO₄, 300 mM NaCl, pH 8.0-8.2). Clarified cell lysates were then added to the column. Thereafter, the resin was then washed with a minimum of 31.5 ml washing buffer prior to elution in 2.5 ml elution buffer (6 M urea, 50 mM NaH₂PO₄, 100 mM NaCl, 30 mM acetic acid, 70 mM Na-acetate, pH 5.0). The eluted material was fractioned in three pools of 500, 700 and 1300 µl. The 700 µl fraction, containing the antigen, and the pooled 500 and 1300 µl fractions were stored for further use.

The antigen fraction was diluted to a final concentration of 1 M urea with phosphate buffered saline (PBS; 1.9 mM NaH₂PO₄, 8.1 mM Na₂HPO₄, 154 mM NaCl) followed by a concentration step to increase the protein concentration using Vivapore 10/20 ml concentrator with molecular weight cut off at 7500 Da (Vivascience AG). The protein concentration was determined using a bicinchoninic acid (BCA) micro assay protocol (Pierce) with a bovine serum albumin standard according to the manufacturer's recommendations. The protein quality was analyzed on a Bioanalyzer instrument using the Protein 50 or 200 assay (Agilent Technologies).

### b) Results

A gene fragment corresponding to nucleotides 434-866 of the long transcript (SEQ ID NO:3) of the WARS gene and encoding a peptide (SEQ ID NO:1) consisting of amino acids 34 to 178 of the target protein WARS (SEQ ID NO:2) was successfully isolated by RT-PCR from a human RNA pool using primers specific for the protein fragment. The 144 amino acid fragment (SEQ ID NO:1) of the target protein (SEQ ID NO:2) was designed to lack transmembrane regions to ensure efficient expression in *E. coli,* and to lack any signal peptide, since those are cleaved off in the mature protein. In addition, the protein fragment was designed to consist of a unique sequence with low homology with other human proteins, to minimize cross reactivity of generated affinity reagents, and to be of a suitable size to allow the formation of conformational epitopes and still allow efficient cloning and expression in bacterial systems.

A clone encoding the correct amino acid sequence was identified, and, upon expression in *E. coli,* a single protein of the correct size was produced and subsequently purified using immobilized metal ion chromatography. After dilution of the eluted sample to a final concentration of 1 M urea and concentration of the sample to 1 ml, the concentration of the protein fragment was determined to be 5.55 mg/ml and was 97.7 % pure according to purity analysis.

### 2. Generation of antibodies

### a) Materials and methods

The purified WARS fragment as obtained above was used as antigen to immunize a rabbit in accordance with the national guidelines (Swedish permit no. A 84-02). The rabbit was immunized intramuscularly with 200 µg of antigen in Freund's complete adjuvant as the primary immunization, and boosted three times in four week intervals with 100 µg antigen in Freund's incomplete adjuvant.

Antiserum from the immunized animal was purified by a three-step immunoaffinity based protocol (Agaton C et al (2004) J. Chromatogr. A 1043:33-40; Nilsson P et al (2005) Proteomics 5:4327-4337). In the first step, 7 ml of total antiserum was buffered with 10x PBS to a final concentration of 1x PBS (1.9 mM NaH₂PO₄, 8.1 mM Na₂HPO₄, 154 mM NaCl), filtered using a 0.45 µm pore-size filter (Acrodisc®, Life Science) and applied to an affinity column containing 5 ml N-hydroxysuccinimide-activated Sepharose™ 4 Fast Flow (GE Healthcare) coupled to the dual affinity tag protein His₆-ABP (a hexahistidyl tag and an albumin binding protein tag) expressed from the pAff8c vector and purified in the same way as described above for the antigen protein fragment. In the second step, the flow-through, depleted of antibodies against the dual affinity tag His₆-ABP, was loaded at a flow rate of 0.5 ml/min on a 1 ml Hi-Trap NHS-activated HP column (GE Healthcare) coupled to the WARS protein fragment used as antigen for immunization (SEQ ID NO:1). The His₆-ABP protein and the protein fragment antigen had been coupled to the NHS activated matrix as recommended by the manufacturer. Unbound material was washed away with 1 x PBST (1 x PBS, 0.1 % Tween20, pH 7.25), and captured antibodies were eluted using a low pH glycine buffer (0.2 M glycine, 1 mM EGTA, pH 2.5). The eluted antibody fraction was collected automatically, and loaded onto two 5 ml HiTrap™ desalting columns (GE Healthcare) connected in series for efficient buffer exchange in the third step. The second and third purification steps were run on the ÄKTAxpress™ platform (GE Healthcare). The antigen selective (mono-specific) antibodies (msAbs) were eluted with PBS buffer, supplemented with glycerol and NaN₃ to final concentrations of 40 % and 0.02 %, respectively, for long term storage at -20 °C (Nilsson P et al (2005) Proteomics 5:4327-4337).

The specificity and selectivity of the affinity purified antibody fraction were analyzed by binding analysis against the antigen itself and against 94 other human protein fragments in a protein array set-up (Nilsson P et al (2005) Proteomics 5:4327-4337). The protein fragments were diluted to 40 µg/ml in 0.1 M urea and 1x PBS (pH 7.4) and 50 µl of each were transferred to the wells of a 96-well spotting plate. The protein fragments were spotted and immobilized onto epoxy slides (SuperEpoxy, TeleChem) using a pin-and-ring arrayer (Affymetrix 427). The slide was washed in 1x PBS (5 min) and the surface was then blocked (SuperBlock®, Pierce) for 30 minutes. An adhesive 16-well silicone mask (Schleicher & Schuell) was applied to the glass before the mono-specific antibodies were added (diluted 1:2000 in 1x PBST to appr. 50 ng/ml) and incubated on a shaker for 60 min. Affinity tag-specific IgY antibodies were co-incubated with the mono-specific antibodies in order to quantify the amount of protein in each spot. The slide was washed with 1x PBST and 1x PBS twice for 10 min each. Secondary antibodies (goat anti-rabbit antibody conjugated with Alexa 647 and goat anti-chicken antibody conjugated with Alexa 555, Molecular Probes) were diluted 1:60000 to 30 ng/ml in 1x PBST and incubated for 60 min. After the same washing procedure, as for the first incubation, the slide was spun dry and scanned (G2565BA array scanner, Agilent), thereafter images were quantified using image analysis software (GenePix 5.1, Axon Instruments).

In addition, the specificity and selectivity of the affinity-purified antibody were analyzed by Western blot. Western blot was performed by separation of total protein extracts from selected human tissues and cell lines on pre-cast 10-20 % SDS-PAGE gradient gels (Bio-Rad Laboratories) under reducing conditions, followed by electro-transfer to PVDF membranes (Bio-Rad Laboratories) according to the manufacturer's recommendations. The membranes were blocked (5 % dry milk, 1x TBST; 0.1 M Tris-HCl, 0.5 M NaCl, 0.1 % Tween20) for 1 h at room temperature, incubated with the primary affinity purified antibody (diluted 1:500 in blocking buffer) and washed in TBST. The secondary HRP-conjugated antibody (swine anti-rabbit immunoglobulin/HRP, DakoCytomation) was diluted 1:3000 in blocking buffer and chemiluminescence detection was carried out using a ChemidoC™ CCD camera (Bio-Rad Laboratories) and SuperSignal® West Dura Extended Duration substrate (Pierce), according to the manufacturer's protocol.

### b) Results

The quality of polyclonal antibody preparations has proven to be dependent on the degree of stringency in the antibody purifications, and it has previously been shown that depletion of antibodies directed against epitopes not originated from the target protein is necessary to avoid cross-reactivity to other proteins and background binding (Agaton C et al (2004) J. Chromatogr. A 1043:33-40).

Thus, a protein microarray analysis was performed to ensure that mono-specific polyclonal antibodies of high specificity had been generated by depletion of antibodies directed against the His₆-tag as well as of antibodies against the ABP-tag. This was followed by affinity capture of antigen selective antibodies on an affinity column with immobilized antigen.

To quantify the amount of protein in each spot of the protein array, a two-color dye labeling system was used, with a combination of primary and secondary antibodies. Tag-specific IgY antibodies generated in hen were detected with a secondary goat anti-hen antibody labeled with Alexa 555 fluorescent dye. The specific binding of the rabbit msAb to its antigen on the array was detected with a fluorescently Alexa 647 labeled goat anti-rabbit antibody. Each protein fragment was spotted in duplicates. The protein array analysis showed that the affinity purified mono-specific antibody against WARS is highly selective to the correct protein fragment and has a very low background to all other protein fragments analyzed on the array.

The result of the Western blot analysis showed that the antibody specifically detects a single band of approximately 50 kDa in a bladder tumor cell line (RT-4) and a human glioma cell line (U-251 MG sp). In addition, a band was detected a in tonsil tissue samples. The theoretical molecular weight of WARS is 49 kDa (as calculated from the WARS amino acid sequence SEQ ID NO:2), corresponding well to the result obtained.

### 3. Tissue profiling by immunohistochemistry

### a) Material and methods

In total, 576 paraffin cores containing human tissues were analyzed using the mono-specific antibody sample obtained in Examples, Section 2. All tissues used as donor blocks for tissue microarray (TMA) production were selected from the archives at the Department of Pathology, University Hospital, Uppsala, in agreement with approval from the local ethical committee. All tissue sections used for TMA analysis were examined to determine diagnosis and to select representative areas in donor blocks. Normal tissue was defined as microscopically normal (non-neoplastic) and was most often selected from specimens collected from the vicinity of surgically removed tumors. Cancer tissue was reviewed for diagnosis and classification. All tissues were formalin fixated, paraffin embedded, and sectioned for diagnostic purposes.

The TMA production was performed essentially as previously described (Kononen J et al (1998) Nature Med. 4:844-847; Kallioniemi OP et al (2001) Hum. Mol. Genet. 10:657-662). Briefly, a hole was made in the recipient TMA block and a cylindrical core tissue sample from the donor block was acquired and deposited in the recipient TMA block. This was repeated in an automated tissue arrayer from Beecher Instrument (ATA-27, Beecher Instruments, Sun Prairie, CA, USA) until a complete TMA design was produced. TMA recipient blocks were baked at 42 °C for 2 h prior to sectioning.

The design of TMA:s was focused on obtaining samples from a large range of representative normal tissues, and on including representative cancer tissues. This has previously been described in detail in Kampf C et al (2004) Clin. Proteomics 1:285-300. In brief, samples from 48 normal tissues and from 20 of the most common cancer types affecting humans were selected. In total, eight different designs of TMA blocks, each containing 72 cores of tissue with 1 mm diameter, were produced. Two of the TMA:s represented normal tissues, corresponding to 48 different normal tissues in triplicates from different individuals. The remaining 6 TMA:s represented cancer tissue from 20 different types of cancer. For 17 of the 20 cancer types, 12 individually different tumors were sampled, and for the remaining 3 cancer types, 4 individually different tumors were sampled, all in duplicates from the same tumor. The TMA blocks were sectioned with 4 µm thickness using a waterfall microtome (Leica), and placed onto SuperFrost® (Roche Applied Science) glass slides for IHC analysis.

Automated immunohistochemistry was performed as previously described (Kampf C et al (2004) Clin. Proteomics 1:285-300). In brief, the glass slides were incubated for 45 min in 60 °C, de-paraffinized in xylene (2 x 15 min) and hydrated in graded alcohols. For antigen retrieval, slides were immersed in TRS (Target Retrieval Solution, pH 6.0, DakoCytomation) and boiled for 4 min at 125 °C in a Decloaking chamber® (Biocare Medical). Slides were placed in the Autostainer® (DakoCytomation) and endogenous peroxidase was initially blocked with H₂O₂ (DakoCytomation). The slides were incubated for 30 min at room temperature with the primary antibody obtained as in Examples, Section 2, followed by incubation for 30 min at room temperature with goat anti-rabbit peroxidase conjugated Envision®. Between all steps, slides were rinsed in wash buffer (DakoCytomation). Finally, diaminobenzidine (DakoCytomation) was used as chromogen and Harris hematoxylin (Sigma-Aldrich) was used for counterstaining. The slides were mounted with Pertex® (Histolab).

All Immunohistochemically stained sections from the eight different TMA:s were scanned using a ScanScope T2 automated slide-scanning systems (Aperio Technologies). In order to represent the total content of the eight TMA:s, 576 digital images were generated. Scanning was performed at 20 times magnification. Digital images were separated and extracted as individual tagged image file format (TIFF) files for storage of original data. In order to be able to handle the images in a web-based annotation system, the individual images were compressed from TIFF format into JPEG format. All images of Immunohistochemically stained tissue were manually evaluated under the microscope and annotated by a board certified pathologist or by specially educated personnel followed by verification of a pathologist.

Annotation of each different normal and cancer tissue was performed using a simplified scheme for classification of IHC outcome. Each tissue was examined for representativity and immunoreactivity. The different tissue specific cell types included in each normal tissue type were annotated. For each cancer, tumor cells and stroma were annotated. Basic annotation parameters included an evaluation of i) subcellular localization (cytoplasmic and/or cytoplasmic/membranous), ii) staining intensity (SI) and iii) fraction of stained cells (FSC). Staining intensity was subjectively evaluated in accordance to standards used in clinical histo-pathological diagnostics and outcome was classified as: absent = no immunoreactivity, weak = faint immunoreactivity, moderate = medium immunoreactivity or strong = distinct and strong immunoreactivity. The fraction of stained cells was estimated and classified as < 2 %, 2 - 25 %, > 25 - 75 % or > 75 % immunoreactive cells of the relevant cell population. Based on both the intensity and fraction of immunoreactive cells, a "staining score" was given for each tissue sample: 0 = negative, 1 = weak, 2 = moderate and 3 = strong. N.R. means that no representative tissues were present. In detail, the staining score was given according to the following criteria: 0 was given if SI = absent or weak and FSC ≤ 25%; 1 was given if SI = weak and FSC > 25% or if SI = moderate and FSC ≤ 25%; 2 was given if SI = moderate and FSC > 25% or if SI = strong and FSC ≤ 25% and SI = moderate; and finally 3 was given if SI = strong and FSC > 25%. See also table 1. The skilled artisan will recognize that this procedure is similar to a calculation of an Allred score, see e.g. Allred *et al* (1998) Mod Pathol 11(2), 155.

| **Table 1:** Staining score | | |
|---|---|---|
| **Staining score** | **Staining intensity** | **Fraction of stained cells** |
| 0 | absent | < 2% |
| 0 | absent | 2 - 25% |
| 0 | absent | > 25 - 75% |
| 0 | absent | > 75% |
| 0 | weak | < 2% |
| 0 | weak | 2 - 25% |
| 1 | weak | > 25 - 75% |
| 1 | weak | > 75% |
| 1 | moderate | < 2% |
| 1 | moderate | 2 - 25% |
| 2 | moderate | > 25 - 75% |
| 2 | moderate | > 75% |
| 2 | strong | < 2% |
| 2 | strong | 2 - 25% |
| 3 | strong | > 25 - 75% |
| 3 | strong | > 75% |

### b) Results

The results from tissue profiling with the mono-specific antibody generated towards a recombinant protein fragment of the human target protein WARS obtained as in Examples, Section 2 showed a particular immunoreactivity in several normal tissues (Table 2) and cancer tissues. Immunoreactivity was observed in cytoplasm. Table 2 shows the WARS protein expression pattern in normal human tissues. Using immunohistochemistry and TMA technology, 144 spots (1 mm in diameter) representing 48 different types of normal tissue were screened for expression of WARS. Strong staining was observed in vessels of the placenta and heart and in macrophages. Otherwise normal tissues generally showed moderate cytoplasmic positivity. Squamous epithelia, muscle tissues and liver were however negative. All other cells and tissues were strong or medium stained. In a few cases no representative tissue (N.R.) were observed.

| **Table 2:** Expression pattern of WARS in normal tissues | | |
|---|---|---|
| **Tissue type** | **Cell type** | **Staining score** |
| **Adrenal gland** | cortical cells | 2 |
| | medullar cells | N.R. |
| **Appendix** | glandular cells | 0 |
| | lymphoid tissue | N.R. |
| **Bone marrow** | bone marrow poetic cells | 2 |
| **Breast** | glandular cells | N.R. |
| **Bronchus** | surface epithelial cells | 2 |
| **Cerebellum** | cells in granular layer | 0 |
| | cells in molecular layer | 0 |
| | purkinje cells | 2 |
| **Cerebral cortex** | neuronal cells | 2 |
| | non-neuronal cells | 1 |
| **Cervix, uterine** | glandular cells | 2 |
| | surface epithelial cells (squamous) | 1 |
| **Colon** | glandular cells | 0 |
| **Duodenum** | glandular cells | 2 |
| **Endometrium** 1 | cells in endometrial stroma/ECM | 2 |
| | cells in myometrium/ECM | 0 |
| | glandular cells | 2 |
| **Endometrium 2** | cells in endometrial stroma/ECM | 0 |
| | cells in myometrium/ECM | 0 |
| | glandular cells | 2 |
| **Epididymis** | glandular cells | 2 |
| **Esophagus** | surface epithelial cells | 0 |
| **Fallopian tube** | glandular cells | 2 |
| **Gall bladder** | glandular cells | 1 |
| **Heart muscle** | myocytes | 0 |
| **Hippocampus** | neuronal cells | 2 |
| | non-neuronal cells | 1 |
| **Kidney** | cells in glomeruli | 1 |
| | cells in tubuli | 1 |
| **Lateral ventricle** | neuronal cells | 2 |
| | non-neuronal cells | 1 |
| **Liver** | bile duct cells | 0 |
| | hepatocytes | 0 |
| **Lung** | alveolar cells | 2 |
| | macrophages | 3 |
| **Lymph node** | follicle cells (cortex) | 1 |
| | non-follicle cells (paracortex) | 1 |
| **Nasopharynx** | surface epithelial cells | 2 |
| **Oral mucosa** | surface epithelial cells | 0 |
| **Ovary** | follicle cells | N.R. |
| | ovarian stromal cells | 0 |
| **Pancreas** | exocrine pancreas | 2 |
| | islet cells | 2 |
| **Parathyroid gland** | glandular cells | 0 |
| **Placenta** | decidual cells | N.R. |
| | trophoblastic cells | 0 |
| **Prostate** | glandular cells | 1 |
| **Rectum** | glandular cells | 1 |
| **Salivary gland** | glandular cells | 2 |
| **Seminal vescicle** | glandular cells | 1 |
| **Skeletal muscle** | myocytes | 0 |
| **Skin** | adnexal cells | N.R. |
| | epidermal cells | 0 |
| **Small intestine** | glandular cells | 1 |
| **Smooth muscle** | smooth muscle cells | 0 |
| **Soft tissue 1** | mesenchymal cells | 2 |
| **Soft tissue 2** | mesenchymal cells | 2 |
| **Spleen** | cells in red pulp | 1 |
| | cells in white pulp | 0 |
| **Stomach 1** | glandular cells | 2 |
| **Stomach 2** | glandular cells | 2 |
| **Testis** | cells in ductus seminiferus | 2 |
| | leydig cells | 2 |
| **Thyroid gland** | glandular cells | 2 |
| **Tonsil** | follicle cells (cortex) | 1 |
| | non-follicle cells (paracortex) | 1 |
| | surface epithelial cells | 0 |
| **Urinary bladder** | surface epithelial cells | 2 |
| **Vagina** | surface epithelial cells | 0 |
| **Vulva/anal skin** | surface epithelial cells | 0 |

WARS protein expression was evaluated in tissue samples from various cancer types. Table 3 shows the level of WARS expression in 12 different colorectal carcinoma tissues samples. All 12 of these samples showed representative tissue, 11 showed positivity and 2 strong expression.

| **Table 3:** Expression pattern of WARS in colorectal cancer | | | | | | | | | | | | |
|---|---|---|---|---|---|---|---|---|---|---|---|---|
| **Cancer type** | **Subject number** | | | | | | | | | | | |
| | **1** | **2** | **3** | **4** | **5** | **6** | **7** | **8** | **9** | **10** | **11** | **12** |
| Colorectal cancer | 3 | 3 | 2 | 2 | 2 | 2 | 2 | 1 | 1 | 1 | 1 | 0 |

### 4) Colorectal cancer TMA

### a) Material and methods

Archival formalin-fixed paraffin-embedded tissue from 122 patients (63 women and 59 men) diagnosed with colorectal carcinoma between 1999 and 2002 was collected from the Department of Pathology, Malmö University Hospital, Sweden. The median age of patients was 75 (32-88) years. 39 tumors were Dukes' stage A, 42 Dukes' stage B and 41 Dukes' stage C. Information regarding the date of death was obtained from the regional cause-of-death registries for all patients. Ethical permission was obtained from the Local Ethics Committee.

All 122 cases of colorectal carcinoma were histopathologically reevaluated on slides stained with hematoxylin and eosin. TMA:s were then constructed by sampling 2 x 1.0 mm cores per case from areas representative of invasive cancer. The TMA:s were prepared and automated immunohistochemistry was performed as described in section 3 above, using the WARS antibody prepared as described in section 2 above.

Tissue annotation was essentially done as described in section 3 above, with the exception that staining intensity and fraction of stained cells were not combined to yield a "staining score". Cytoplasmic intensity and cytoplasmic fraction were instead analyzed separately for correlation with survival. "cytoplasmic intensity" (CI) corresponds to the overall staining intensity of cytoplasms of glandular/tumor cells in a sample. "Cytoplasmic fraction" (CF) corresponds to the percentage of glandular/tumor cells in a sample that exhibits a positive staining intensity in the cytoplasm.

Based on the survival trends for all different strata, a dichotomized variable was constructed for further statistical analyses. From this analysis, the cytoplasmic intensity was divided into: absent cytoplasmic intensity (0); or weak, moderate or strong cytoplasmic intensity (> 0). Further, the cytoplasmic fraction was divided into: < 2 % positive cells (0), i.e. less than 2 % of the cells in a sample exhibiting a positive staining in the cytoplasm; or ≥ 2 % positive cells (1-3), i.e. 2 % or more of the cells in a sample exhibiting a positive staining in the cytoplasm.

The above classification of samples was used for overall survival analysis according to the Kaplan-Meier method, and the log-rank test was used to compare survival in different strata. All statistical tests were two- sided, and p-values of < 0.05 % were considered significant. All calculations were made with the statistical package SPSS 12.0 (SPSS Inc. Illinois, USA).

### b) Results

Tissue microarray based analysis of 122 colorectal carcinomas showed that 78 subjects (64 %) were positive for WARS (CF ≥ 2 %) as seen in figure 1A. The figure further reveals that for 58% of the subjects, fractions of stained cells are > 25%. The intensity was in 67% of the subjects weak, moderate or strong, as seen in figure 1B. Survival analysis based on the cytoplasmic fraction or cytoplasmic intensity for the entire cohort revealed a significantly (p < 0.03) lower five year overall survival (OS) for patients having tumors with no WARS expression (figure 2A and 2B). With regard to cytoplasmic fraction, the overall five-year survival was about 50 % for patients with less than 2 % of the cells positive for WARS (CF = 0) and about 70 % for patients with 2 % or more of the cells positive for WARS (CF > 0) (figure 2A). A similar trend is observed for cytoplasmic intensity: the overall five-year survival was about 70 % for patients with a weak, moderate or strong cytoplasmic intensity (CI > 0) and about 45 % for patients with an absent cytoplasmic intensity (CI = 0) (figure 2B).

Further, when looking at subjects having dukes' A colorectal cancers only, the trend is consistent with the data presented above: the overall five-year survival was about 80 % for patients with a weak, moderate or strong cytoplasmic intensity (CI > 0) and about 48 % for patients with an absent cytoplasmic intensity (CI = 0) (figure 3).

As can be seen from figures 2 and 3, in a patient group diagnosed with colorectal cancer, the level of WARS protein expression clearly correlates with probability of survival, such as five-year survival.

### Establishment of a prognosis for a colorectal cancer patient

### 5. A non-limiting example

A cancer patient can present symptoms or signs from a tumor growth, focal symptoms including pain and distress from the region where the tumor grows or more general symptoms such as weight loss and fatigue. Signs from growth of a colorectal tumor can also become evident through blood in feces and/or dysfunction, e.g. diarrhea/constipation.

Following the establishment of a colorectal cancer diagnosis in a patient, a tumor tissue sample is obtained.The tumor tissue sample may be obtained from a biopsy performed earlier during the diagnosis of the cancer or from a specimen from an earlier surgical removal of the tumor. Further, for the provision of a "negative reference", a sample is taken from archival material comprising tissue having low, or essentially lacking, WARS protein expression. Such archival tissue may for example be colorectal cancer tissue having a pre-established low WARS protein expression level or an appropriate tissue having a staining score of 0 in Table 2, such as myocytes from skeletal muscle. Further, for the provision of a "positive reference", a sample is taken from archival material comprising tissue having high WARS protein expression, such as colorectal cancer tissue having a pre-established high WARS protein expression level or an appropriate tissue having a staining score of 2 in Table 2, such as normal urothelial tissue.

The sample material is fixated in buffered formalin and histo-processed in order to obtain thin sections (4 µm) of the of the sample material.

Immunohistochemistry is performed as described in Examples, Section 3. One or more sample sections from each sample are mounted on glass slides that are incubated for 45 min in 60 °C, de-paraffinized in xylene (2 x 15 min) and hydrated in graded alcohols. For antigen retrieval, slides are immersed in TRS (Target Retrieval Solution, pH 6.0, DakoCytomation) and boiled for 4 min at 125 °C in a Decloaking chamber® (Biocare Medical). Slides are placed in the Autostainer® (DakoCytomation) and endogenous peroxidase is initially blocked with H₂O₂ (DakoCytomation). The reason for mounting multiple sample sections may be to increase the accuracy of the results.

A primary WARS specific antibody is added to the slides and incubated for 30 min in room temperature, followed by a 30 min incubation in room temperature with a labeled secondary antibody; e.g. goat-anti-rabbit peroxidase conjugated Envision®. To detect the secondary antibody, diaminobenzidine (DakoCytomation) is used as chromogen, contrasted with a Harris hematoxylin (Sigma-Aldrich) counterstaining. Between all steps, slides are rinsed in wash buffer (DakoCytomation). The slides are then mounted with Pertex® (Histolab) mounting media.

As a tool to validate the staining procedure, two control cell-lines may be used; e.g. one slide with cells expressing WARS (positive cell line) and one slide having cells with indistinct weak or no WARS expression (negative cell line). The skilled artisan understands how to provide such cell lines, for example guided by the disclosure of Rhodes et al. (2006) The biomedical scientist, p 515-520. The control-line slides may be simultaneously stained in the same procedure as the colorectal cancer slides, i.e. incubated with the same primary and secondary antibodies.

For example, the colorectal cancer tumor slides, the staining reference slides, and optionally, the slides with control cell-lines, may be scanned in a light microscope using a ScanScope T2 automated slide scanning system (Aperio Technologies) at x20 magnification.

If control cell-lines are used, these are inspected to validate the staining procedure. If the cell-lines display staining results outside acceptable criteria, e.g. staining artifacts recognized by the skilled artisan, the staining of the biopsy samples is considered invalid and the whole staining procedure is repeated with new slides. If the positive and negative cell-lines display strong staining intensity and indistinct weak or no staining intensity, respectively, the staining is considered as valid.

The stained sample slide(s) from the tumor tissue biopsy is/are evaluated manually by visual inspection in accordance to standards used in clinical histo-pathological diagnostics, and the immunoreactivity of the colorectal cancer slide(s) is/are graded as described in Examples, Section 4.

That is, the cytoplasmic intensity (CI) and/or the cytoplasmic fraction (CF) is determined. CI corresponds to the overall staining intensity of cytoplasms of cells in a sample and is divided into: absent cytoplasmic intensity (CI = 0); or weak, moderate or strong cytoplasmic intensity (CI > 0). CF corresponds to the percentage of cells in a sample that exhibits a positive staining intensity in the cytoplasm and is divided into: < 2 % positive cells (CF = 0), i.e. if less than 2 % of the cells in a sample exhibit a positive staining; or ≥ 2 % positive cells (CF > 0), i.e. if 2 % or more of the cells in a sample exhibit a positive staining.

In the determination of the Cl and/or the CF, the person performing the evaluation and grading is aided by visual inspection of the stained reference slides, i.e. the "positive reference" and the "negative reference".

The sample value(s), i.e. the CI(s) and/or the CF(s), of the sample slide(s) from the tumor tissue biopsy are then compared to a reference value. The reference value may for example be an absent CI (no immunoreactivity) and/or a CF < 2 %. If the sample value(s) are equal to or lower than the reference value, a conclusion is drawn that the prognosis is worse than or equal to a reference prognosis being associated with the reference value. For example, if the reference value is an absent CI, which may be associated with a probability of five-year survival of lower than 70 %, the conclusion may be that the prognosis for the patient is a probability of five-year survival of lower than 70 %. Also, if the reference value CF < 2 %, which may be associated with a probability of five-year survival of lower than 66 %, the conclusion may be that the prognosis for the patient is a probability of five-year survival of less than 70 %. The prognosis may then form a basis for further decisions relating to the treatment, or non-treatment, of the patient.

## Claims

1. Method for determining whether a prognosis for a mammalian subject having a colorectal cancer is worse than or equal to a reference prognosis, comprising the steps of:
a) providing a sample earlier obtained from the subject;
b) evaluating the amount of WARS protein present in at least part of said sample, and determining a sample value corresponding to said evaluated amount;
c) comparing the sample value obtained in step b) with a reference value associated with said reference prognosis; and, if said sample value is equal to or lower than said reference value,
d) concluding that the prognosis for said subject is equal to or worse than said reference prognosis.

2. Method according to claim 1, wherein the sample is a body fluid sample.

3. Method according to claim 2, wherein the body fluid is selected from the group consisting of blood, plasma, serum, cerebral fluid, urine, semen and exudate.

4. Method according to claim 1, wherein said sample is a tissue sample.

5. Method according to claim 4, wherein the tissue sample is a colorectal tissue sample.

6. Method according to claim 1, wherein the sample is a cytology sample.

7. Method according to claim 1, wherein the sample is a stool sample.

8. Method according to any preceding claim, wherein said sample comprises tumor cells from said subject.

9. Method according to any preceding claim, wherein the evaluation of step b) is limited to evaluating the amount of WARS expression in the cytoplasm of tumor cells of said sample.

10. Method according to any preceding claim, wherein said colorectal cancer is in Dukes' stage A or B.

11. Method according to any preceding claim, wherein said colorectal cancer is in Dukes' stage A.

12. Method according any preceding claim, wherein said prognosis for said subject is a probability of survival and said reference prognosis is a probability of survival.

13. Method according to any preceding claim, wherein said reference value is a predetermined value corresponding to the amount of WARS protein expression in a reference sample.

14. Method according to any preceding claim, wherein the sample value of step b) is determined as being either 1, corresponding to detectable WARS protein in the sample, or 0, corresponding to no detectable WARS protein in the sample.

15. Method according to any preceding claim, wherein the reference value of step c) corresponds to a reference sample having no detectable WARS protein.

16. Method according to any preceding claim, wherein the reference value of step c) is 0.

17. Method according to any preceding claim, wherein said reference value is a cytoplasmic fraction, a cytoplasmic intensity or a combination thereof.

18. Method according to claim 17, wherein said reference value is a cytoplasmic fraction of 25 % WARS positive cells, or lower.

19. Method according to claim 17 wherein said reference value is a weak cytoplasmic intensity of WARS protein expression, or lower.

20. Method according to any preceding claim, wherein said subject is a human.

21. Method according to any preceding claim, wherein the amino acid sequence of the WARS protein comprises a sequence selected from:
i) SEQ ID NO:1; and
ii) a sequence which is at least 85 % identical to SEQ ID NO:1.

22. Method according to any preceding claim, wherein the amino acid sequence of the WARS protein comprises a sequence selected from:
i) SEQ ID NO:2; and
ii) a sequence which is at least 85 % identical to SEQ ID NO:2.

23. Method according to any preceding claim, wherein step b) comprises:
b1) applying to the sample a quantifiable affinity ligand capable of selective interaction with the WARS protein to be evaluated, said application being performed under conditions that enable binding of the affinity ligand to any WARS protein present in the sample;
b2) removing non-bound affinity ligand; and
b3) quantifying any affinity ligand remaining in association with the sample to evaluate said amount.

24. Method according to claim 23, wherein the quantifiable affinity ligand is selected from the group consisting of antibodies, fragments thereof and derivatives thereof.

25. Method according to any one of claims 23-24, wherein the quantifiable affinity ligand is capable of selective interaction with a WARS protein having the sequence of SEQ ID NO:1.

26. Method according to any one of claims 23-25, wherein said quantifiable affinity ligand is detected using a secondary affinity ligand capable of recognizing the quantifiable affinity ligand.

27. Method according to claim 26, wherein said secondary affinity ligand capable of recognizing the quantifiable affinity ligand comprises a label selected from the group consisting of fluorescent dyes and metals, chromophoric dyes, chemiluminescent compounds and bioluminescent proteins, enzymes, radioisotopes, particles and quantum dots.

28. Method according to any preceding claim, wherein said reference prognosis is a five-year survival probability of less than 70 %.

29. Method according to any one of claims 1-28, which, if said sample value is higher than said reference value, further comprises a step of:
e) concluding that the prognosis for said subject is better than said reference prognosis.

30. Kit for carrying out the method according to any preceding claim, which comprises
a) a quantifiable affinity ligand capable of selective interaction with a WARS protein; and
b) reagents necessary for quantifying the amount of the affinity ligand.

31. Kit according to claim 30, in which the quantifiable affinity ligand is selected from the group consisting of antibodies, fragments thereof and derivatives thereof.

32. Kit according to claim 31, in which the quantifiable affinity ligand is obtainable by a process comprising a step of immunizing an animal with a protein whose amino acid sequence comprises the sequence SEQ ID NO:1.

33. Kit according to any one of claims 30-32, in which the quantifiable affinity ligand is capable of selective interaction with a polypeptide having the sequence SEQ ID NO:1.

34. Kit according to any one of claims 30-33, in which said reagents necessary for quantifying the amount of the affinity ligand comprise a secondary affinity ligand capable of recognizing the quantifiable affinity ligand.

35. Kit according to claim 34, in which said secondary affinity ligand capable of recognizing the quantifiable affinity ligand comprises a label selected from the group consisting of fluorescent dyes or metals, chromophoric dyes, chemiluminescent compounds and bioluminescent proteins, enzymes, radioisotopes, particles and quantum dots.

36. Kit according to any one of claims 30-35, further comprising at least one reference sample for provision of a reference value.

37. Kit according to claim 36, in which at least one reference sample is a tissue sample having no detectable WARS protein present.

38. Kit according to any one of claims 36-37, in which at least one reference sample comprises WARS protein.

39. Kit according to claim 38, in which at least one reference sample comprises an amount of WARS protein corresponding to a cytoplasmic fraction of 25 % WARS positive cells or lower.

40. Kit according to claim 38, in which at least one reference sample comprises an amount of WARS protein corresponding to a weak cytoplasmic intensity of WARS protein expression or lower.

41. Kit according to any one of claims 36-40, in which at least one reference sample contains an amount of WARS protein corresponding to a value being higher than the reference value.

42. Kit according to any one of claims 36-41 comprising:
a first reference sample comprising an amount of WARS protein being higher than the reference value; and
a second reference sample comprising an amount of WARS protein being lower than the reference value.

43. Kit according to any one of claims 36-42, in which the reference sample(s) is/are tissue sample(s).

44. Use of a WARS protein as a prognostic marker.

45. Use of a WARS protein as a prognostic marker for cancer.

46. Use according to claim 45, wherein said cancer is a colorectal cancer.

47. Use of a WARS protein, or an antigenically active fragment thereof, for the production, selection or purification of a prognostic agent for establishing a prognosis for a patient having a colorectal cancer.

48. Use according to claim 47, wherein said prognostic agent is an affinity ligand capable of selective interaction with the WARS protein, or an antigenically active fragment thereof.

49. Use according to claim 48, wherein the affinity ligand is capable of selective interaction with a protein having the sequence SEQ ID NO:1.

50. Use according any one of claims 44-49, wherein the amino acid sequence of the WARS protein comprises a sequence selected from:
i) SEQ ID NO:1; and
ii) a sequence which is at least 85 % identical to SEQ ID NO:1.

51. Use according any one of claims 44-50, wherein the amino acid sequence of the WARS protein comprises a sequence selected from:
i) SEQ ID NO:2; and
ii) a sequence which is at least 85 % identical to SEQ ID NO:2.

52. Affinity ligand capable of selective interaction with a WARS protein, which is an antibody or a fragment or a derivative thereof.

53. Affinity ligand according to claim 52, which is obtainable by a process comprising a step of immunizing an animal with a protein whose amino acid sequence comprises the sequence SEQ ID NO:1.

54. Affinity ligand according to claim 53, which is obtainable by a process comprising a step of immunizing an animal with a polypeptide having the sequence SEQ ID NO:1.

55. Affinity ligand according to any one of claims 52-54, capable of selective interaction with a polypeptide having the sequence SEQ ID NO:1.

56. Affinity ligand according to any one of claims 52-55 for evaluating the amount of WARS protein present in at least part of a sample obtained from a subject having a colorectal cancer.

57. Affinity ligand according to any one of claims 52-56 for establishing a prognosis for a mammalian subject having a colorectal cancer.

58. Use of an affinity ligand according to any one of claims 52-57 as a prognostic agent.

59. Use of an affinity ligand according to any one of claims 52-57 for establishing a prognosis for a mammalian subject having a colorectal cancer.
